# EUROPEAN PATENT SPECIFICATION

(11) **EP 1 592 648 B1**
(45) Date of publication and mention of the grant of the patent: **24.01.2007**
(21) Application number: 04707716.9
(22) Date of filing: 03.02.2004
(51) Int. Cl.: C07C 2/64, C07C 15/107

(54) **METHOD OF PREPARING BRANCHED ALKYL AROMATIC HYDROCARBONS USING A PROCESS STREAM FROM A DEHYDROGENATION-ISOMERIZATION UNIT**
METHODE ZUR HERSTELLUNG VON VERZWEIGTEN ALKYLSUBSTITUIERTEN AROMATISCHE KOHLEWASSERSTOFFEN UNTER VERWENDUNG EINES PROZESSSTROMES AUS EINER DEHYDRIERUNGS-ISOMERISIERUNGSEINHEIT
PROCEDE POUR PREPARER DES HYDROCARBURES ALKYLE AROMATIQUES RAMIFIES AU MOYEN D'UN FLUX DE SUBSTANCES A TRAITER A PARTIR D'UNE UNITE DE DESHYDROGENATION-ISOMERISATION

(30) Priority: 05.02.2003 US 445299 P
(43) Date of publication of application: 09.11.2005
(73) Proprietor: SHELL INTERNATIONALE RESEARCH MAATSCHAPPIJ B.V., 2596 HR Den Haag (NL)
(72) Inventor: AYOUB, Paul, Marie, NL-1031 CM Amsterdam (NL); DIRKZWAGER, Hendrik, NL-1031 CM Amsterdam (NL); MURRAY, Brendan, Dermot, Houston, TX 77077 (US); SUMROW, Steven, Clois, Katy, TX 77450 (US)
(86) International application number: PCT/US2004/002956
(87) International publication number: WO 2004/071999

(56) References cited:
- WO-A-02/44114
- US-A- 3 492 364

## Description

### BACKGROUND OF THE INVENTION

### 1. Field of Invention

The present invention generally relates to systems and methods for preparing alkyl aromatic hydrocarbons. More particularly, embodiments described herein relate to systems and methods for preparing branched alkyl aromatic hydrocarbons.

### 2. Description of Related Art

Alkylated aromatic hydrocarbons are important compounds that may be used in a variety of applications or converted to other chemical compounds (e.g. surfactants, sulfonates). Surfactants may be used in a variety of applications, for example detergents, soaps and oil recovery.

The structural composition of an alkyl aromatic hydrocarbon may influence the properties (e.g., water solubility, biodegradability, cold water detergency) of the surfactant and/or detergent produced from an alkyl aromatic hydrocarbon. For example, water solubility may be affected by linearity of the alkyl group. As the linearity of the alkyl group increases, the hydrophilicity (i.e., affinity for water) of the alkyl aromatic surfactant may decrease. Thus, the water solubility and/or detergency (performance) of the alkyl aromatic surfactant may decrease. Incorporating branches that contain a minimum number of quaternary and/or tertiary carbon atoms into the alkyl portion of the alkyl aromatic surfactant may increase the cold-water solubility and/or detergency of the alkyl aromatic surfactant while maintaining the biodegradability of a detergent. The amount and type of branching of the alkyl group, however, may decrease the biodegradation rate of a surfactant and/or detergent.

Branched alkyl aromatic hydrocarbons are composed of a branched alkyl group coupled to an aromatic group. Branched alkyl groups are composed of a linear alkyl groups with branches extending form the linear alkyl group. Branches of the linear alkyl groups may include one or more aliphatic alkyl groups, a linear alkyl group or combinations thereof. Branches may include methyl, ethyl, propyl or higher alkyl groups. Quaternary and tertiary carbons may be present in a branched the alkyl group. The number of quaternary and tertiary carbons may result from the branching pattern in the alkyl group. As used herein, the phrase "aliphatic quaternary carbon atom" refers to a carbon atom that is not bound to any hydrogen atoms and not attached an aromatic ring system.

A surfactant with a branched alkyl group including quaternary and/or tertiary carbons may have a lower biodegradation rate than a surfactant with a linear or mono-branched alkyl group. As used herein, "biodegradable" refers to a material that can be chemically altered or broken down by bacteria or other natural agents. For example, in a biodegradation experiment using a porous pot activated sludge treatment, a biodegradation rate of sodium 2-methyl-2-undecyl[¹⁴C]benzensulfonate was greater than a biodegradation rate of sodium 5-methyl-5-undecyl[¹⁴C]benzensulfonate, A detailed description of the experiment is described by Nielsen et al. in "Biodegradation of Coproducts of Commercial Linear Alkylbenzene Sulfonate," *Environmental Science and Technology*, 1997, 31:3397-3404.

Examples of compositions and processes for the manufacture of branched alkyl aromatic hydrocarbons are described in U.S. Patent No. 3,484,498 to Berg, entitled "Process For The Preparation Of Aryl-Substituted Normal Paraffin Hydrocarbons"; U.S. Patent No. 6,111,158 to Marinangeli et al., entitled "Process For Producing Arylalkanes At Alkylation Conditions Using A Zeolite Having a NES Zeolite Structure Type" and U. S. Patent No. 6,187,981 to Marinangeli et al., entitled "Process For Producing Arylalkanes And Arylalkane Sulfonates, Compositions Produced Therefrom, and Uses Thereof".

More recently, it has been found that surfactants derived from branched olefins have different, more desirable properties than surfactants derived from linear olefins. For example, surfactants derived from branched olefins have increased water solubility, improved detergency properties and acceptable biodegradable properties compared to surfactants derived from linear olefins. Production of branched olefins from a Fischer-Tropsch process stream may increase an economic value of the stream. In some embodiments, linear olefins may be converted into branched olefins using a dehydrogenation-isomerization catalyst. The branched olefins may be used to produce surfactants that are more desirable and thus more valuable to the producer of the Fischer-Tropsch stream. In general, Fischer-Tropsch processes tend to produce minor amounts of olefins. Increasing an olefin content (e.g. branched alkyl olefin content) of a Fischer-Tropsch stream may increase the economic value of the stream.

WO 02/44114 A1 discloses a method for the production of alkyl Akyl Sulfonates.

### SUMMARY

In an embodiment, a feed stream that includes olefins and paraffins may be processed in a dehydrogenation-isomerization unit. The process feed stream entering a dehydrogenation-isomerization unit, in some embodiments, is derived from a Fischer-Tropsch process. In the dehydrogenation-isomerization unit at least a portion of the paraffins in the feed stream may be dehydrogenated to form olefins. The dehydrogenation-isomerization unit may also isomerize at least a portion of the resulting olefins and at least a portion of the olefins that were already present in the feed stream. The isomerization process converts at least a portion of linear olefins (i.e., unbranched olefins) into branched olefins.

Process conditions in the dehydrogenation-isomerization unit may be such that the resulting branched olefins have an average number of branches per olefin molecule of between about 0.7 and about 2.5. The branched olefin may include, but is not limited to methyl and/or ethyl branches. The isomerization process may produce branched olefins that include less than about 0.5 percent of quaternary aliphatic carbon atoms. Process conditions in the dehydrogenation-isomerization unit may include a catalyst that has two functions, one to dehydrogenate the paraffins to olefins and the second to isomerize the olefins.

In an embodiment, a dehydrogenation-isomerization unit may include a plurality of zones. The plurality of zones may include a first reaction zone and a second reaction zone. The first reaction zone may be a dehydrogenation zone. The second reaction zone may be an isomerization zone. A hydrocarbon stream containing olefins and paraffins may enter the dehydrogenation zone. At least a portion of the paraffins in the hydrocarbon stream may be dehydrogenated to olefins to produce an olefin enriched stream. The enriched olefin stream may be passed into the isomerization zone. In the isomerization zone, at least a portion of the olefins in the enriched olefin stream may be isomerized to branched olefins. The branched olefins may be used to alkylate aromatic hydrocarbons.

In an embodiment, one or more hydrocarbons streams may be combined with a feed stream entering the dehydrogenation-isomerization unit. The hydrocarbons stream may be mixed with the feed stream to alter the concentration of the olefins entering the dehydrogenation-isomerization unit After the feed stream is processed in the dehydrogenation-isomerization unit, the resulting branched olefin-containing stream is passed into an alkylation unit. One or more hydrocarbons streams may be combined with the branched olefin-containing stream to alter the concentration of olefins entering the alkylation unit. In the alkylation unit, at least a portion of aromatic hydrocarbons may be alkylated with at least a portion of the olefins in the combined stream.

Alkylation of aromatic hydrocarbons with olefins may occur in an alkylation unit. In an embodiment, an olefinic hydrocarbons stream from a dehydrogenation-isomerization unit and aromatic hydrocarbons may enter an alkylation unit. In the alkylation unit, at least a portion of the aromatic hydrocarbons may be alkylated with at least a portion of the olefins in the hydrocarbon stream to produce alkyl aromatic hydrocarbons. At least a portion of the produced alkyl aromatic hydrocarbons may include a branched alkyl group. At least a portion of the unreacted components of the hydrocarbons stream, at least a portion of unreacted aromatic hydrocarbons and at least a portion of the produced alkyl aromatic hydrocarbons may form an alkylation reaction stream.

After alkylation of the aromatic hydrocarbons, at least a portion of the paraffins and unreacted olefins and aromatic hydrocarbons from the alkylation process may be separated from the alkyl aromatic hydrocarbon products. The paraffins and unreacted olefins may be separated from the aromatic hydrocarbons to form a paraffins and unreacted olefins stream. The paraffins and unreacted olefins stream may recycle by directing the paraffins and unreacted olefins stream back into the dehydrogenation-isomerization unit and/or into a stream entering the dehydrogenation-isomerization unit. The aromatic hydrocarbons may be recycled back to the alkylation unit.

In certain embodiments, at least a portion of an alkyl aromatic hydrocarbons product stream may be sulfonated to form alkyl aromatic sulfonates. In some embodiments, the alkyl aromatic sulfonates may include branched alkyl groups.

### BRIEF DESCRIPTION OF THE DRAWINGS

Advantages of the present invention will become apparent to those skilled in the art with the benefit of the following detailed description of embodiments and upon reference to the accompanying drawings, in which:
FIG. 1 depicts a schematic diagram of an embodiment of a system for producing branched alkyl aromatic hydrocarbons using a dehydrogenation-isomerization unit.
FIGS. 2 A-B depict schematic diagrams of embodiments of a system for producing branched alkyl aromatic hydrocarbons using a two-zone dehydrogenation-isomerization unit.
FIG. 3 depicts a schematic diagram of an embodiment of a system for producing branched alkyl aromatic hydrocarbons using a dehydrogenation-isomerization unit with a stacked bed catalyst configuration.

While the invention is susceptible to various modifications and alternative forms, specific embodiments thereof are shown by way of example in the drawing and will herein be described in detail. It should be understood, however, that the drawings and detailed description thereto are not intended to limit the invention to the particular form disclosed, but on the contrary, the intention is to cover all modifications, equivalents, and alternatives falling within the spirit and scope of the present invention as defined by the appended claims.

### DETAILED DESCRIPTION OF EMBODIMENTS

Hydrocarbon products may be synthesized from synthesis gas (i.e., a mixture of hydrogen and carbon monoxide) using a Fischer-Tropsch process. Synthesis gas may be derived from coal or by reforming of natural gas. The Fischer-Tropsch process catalytically converts synthesis gas into a mixture of products that includes primarily saturated hydrocarbons, a certain amount of olefins and a minor amount of oxygen containing products. The products from a Fischer-Tropsch process may be used for the production of fuels (e.g., gasoline, diesel oil), lubricating oils and waxes. Fuels and other products produced by a Fischer-Tropsch process generally have low levels of sulfur, nitrogen and/or metals and contain little or no cyclic compounds (e.g., (aromatics, naphthalenes).

Fischer-Tropsch process streams may also be used to prepare economically valuable commodity products. For example, linear olefins are commodity products that are useful for the production of surfactants. Using a portion of the process stream to produce linear olefins may increase the economic value of a Fischer-Tropsch process stream. To reduce production costs of producing alkyl aromatic hydrocarbons, a stream containing a significant amount of paraffins and a minor amount of olefins may first be dehydrogenated and isomerized, then alkylated to form alkyl aromatic hydrocarbons. Processing the low olefin feed stream through a dehydrogenation-isomerization unit prior to alkylation may save production time, catalyst cost, and/or enhance the overall economic viability of the low olefin process stream.

A hydrocarbon feed stream composition may include paraffins and olefins. At least a portion of the hydrocarbon stream may be made up of linear paraffins and olefins having at least 4 carbon atoms and up to 24 carbon atoms. A hydrocarbon feed stream may be obtained from a Fischer-Tropsch process or from an ethylene oligomerization process. Fischer-Tropsch catalysts and reaction conditions may be selected to provide a particular mix of products in the reaction product stream. Catalysts and/or combinations of catalyst that favor the manufacture of desired hydrocarbon species in a Fischer-Tropsch process are generally known.

While reference is made to a Fischer-Tropsch stream, it is to be understood that any stream, made by any process, that includes olefins and saturated hydrocarbons may be a suitable feedstock for the processes disclosed herein. Many Fischer-Tropsch streams may contain from 5 percent to 99 percent olefins, the remainder being saturated hydrocarbons and other compounds.

In some embodiments, feed streams containing olefins and paraffins are obtained through cracking of paraffin wax or the oligomerization of olefins. Commercial olefin products manufactured by ethylene oligomerization are marketed in the United States by Chevron Chemical Company, Shell Chemical Company (under the trademark NEODENE), and by British Petroleum. Cracking of paraffin wax to produce alpha-olefin and paraffin feed streams is described in U.S. Patent No. 4,579,986 to Sie, entitled "Process For The Preparation Of Hydrocarbons" and U.S. Patent Application Serial No. 10/153,955 to Ansorge et al., entitled "Process For The Preparation of linear Olefins and Use Thereof To Prepare Linear Alcohols". Specific procedures for preparing linear olefins from ethylene are described in U.S. Patent No. 3,676,523 to Mason, entitled "Alpha-Olefin Production"; U.S. Patent No. 3,686,351 to Mason, entitled "Alpha-Olefin Production; "U.S. Patent No. 3,737,475 to Mason, entitled "Alpha-Olefin Production" and U. S. Patent No. 4,020,121 to Kister et al., entitled "Oligomerization Reaction System." Most of the above-mentioned processes produce alpha-olefins. Higher linear internal olefins may be commercially produced, for example, by the chlorination-dehydrochlorination of paraffins, by paraffin dehydrogenation, or by isomerization of alpha-olefins.

In an embodiment, a feed stream is processed to produce a hydrocarbon stream that includes branched olefins. Branched olefins may be used to alkylate an aromatic hydrocarbon to produce branched alkyl aromatic hydrocarbons. The feed stream may have a paraffin content range between about 50 percent by weight and about 90 percent by weight of the feed stream. In certain embodiments, a feed stream may have a paraffin content greater than about 90 percent by weight paraffins. The feed stream may also include olefins. The olefin content of the feed stream may be between about 10 percent by weight and about 50 percent by weight. In other embodiments, a feed stream may have an olefin content greater than 90 percent by weight olefins. The composition of the feed stream may include hydrocarbons having an average carbon number ranging from 4 to 30. In certain embodiments, an average carbon number of the hydrocarbons in a feed stream may range from 4 to 24, from 4 to 16, or from 10 to 13. A feed stream may include minor amounts of hydrocarbons having a carbon number that is higher or lower than the desired carbon number range. In some embodiments, a feed stream may be derived from distillation of a process stream that includes a broader range of carbon numbers.

In an embodiment, a feed stream for a dehydrogenation-isomerization unit includes mono-olefins and/or paraffins. The mono-olefins may be of a linear or branched structure. The mono-olefins may have an alpha or internal double bond. The feed stream may include olefins in which 50 percent or more of the olefin molecules present may be alpha-olefins of a linear (straight chain) carbon skeletal structure. In certain embodiments, at least about 70 percent of the olefins are alpha-olefins of a linear carbon skeletal structure. A hydrocarbon stream in which greater than about 70 percent of all of the olefin molecules are alpha-olefins of a linear carbon skeletal structure may be used in certain embodiments of alkylation of aromatic hydrocarbons. Such a stream may be derived from a Fischer-Tropsch process. In some embodiments, a feed stream includes olefins in which at least about 50 percent of the olefin molecules present are internal olefins.

Branched chain olefins may be used as a feed source for an aromatic alkylation reaction. An aromatic alkylation reaction uses olefins in an incoming stream to alkylate aromatic compounds. Aromatic compounds (e.g., aromatic hydrocarbons) may include benzene or substituted benzene. In an embodiment, alkyl-substituted benzene is used as a substituted benzene in the process. Alkyl-substituted benzenes may be mono- and/or polysubstituted lower alkyl benzenes. The alkyl substituent of an alkyl substituted benzene may have a carbon number ranging from 1 to 5. In an embodiment, a carbon number of the alkyl substituent may range from 1 to 2. Suitable examples of aromatic compounds include, but are not limited to, benzene, toluene, xylenes, ethylbenzene, cumene, n-propylbenzene and other mono- and poly-lower alkylbenzenes. In some embodiments, a single aromatic compound or a mixture of two or more aromatic compounds may be used as feed source. The aromatic hydrocarbons may be fed into the reactor directly or mixed in a suitable non-reactive organic solvent prior to addition to the alkylation unit.

A unit that combines the processes of dehydrogenation and isomerization in the production of branched olefins from a predominately paraffinic feed stream may be desired. By combining two processes into one unit, a more economically valuable process may result (e.g., greater utilization of a mostly paraffinic hydrocarbon stream). Processes using one unit to produce branched olefins with a substantially minimal amount quaternary and/or tertiary carbon atoms are described. The resulting branched olefins may be converted to alkyl aromatic hydrocarbons, which may be used to manufacture other commercially valuable products (e.g., surfactants).

In certain embodiments, a first hydrocarbon stream that includes paraffins and olefins may be introduced into a dehydrogenation-isomerization unit. The dehydrogenation-isomerization unit may replace two independent units (e.g., an isomerization unit and a dehydrogenation unit). The dehydrogenation-isomerization unit may dehydrogenate paraffins to olefins and isomerize the resulting olefins and/or initial olefins present in the hydrocarbon stream to branched olefins. In an embodiment, a catalyst may promote the dehydrogenation-isomerization of hydrocarbons in the first hydrocarbon stream. In certain embodiments, a catalyst may be a single catalyst. A catalyst, in some embodiments, may be a mixture of two catalysts (e.g., a dehydrogenation catalyst and an isomerization catalyst). In other embodiments, two separate catalysts located in different zones or in a stacked bed configuration in one dehydrogenation-isomerization unit may perform the dehydrogenation-isomerization process. As used herein, "a dehydrogenation-isomerization catalyst" may be one or more catalysts.

In certain embodiments, a dehydrogenation-isomerization unit may have several points of entry to accommodate different process streams. The process streams may be from other processing units and/or storage units. Examples of process streams include, but are not limited to, a diluent hydrocarbon stream, and/or other hydrocarbon streams that include olefins and paraffins derived from other processes. As used herein "entry into the dehydrogenation-isomerization unit" refers to entry of process streams into the dehydrogenation-isomerization unit through one or more entry points.

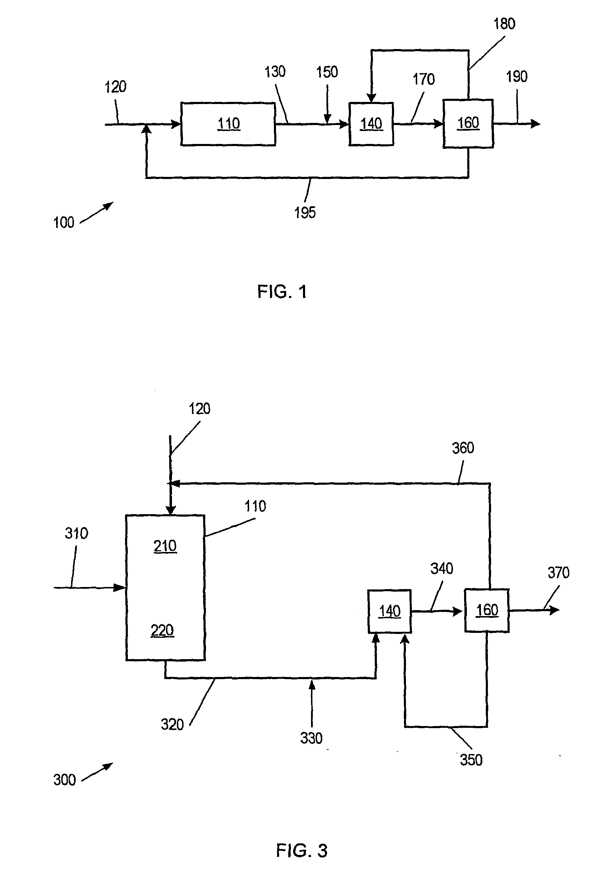

Referring to System 100, as depicted in FIG. 1, a first hydrocarbon stream, including a mixture of olefins and paraffins, may be introduced into dehydrogenation-isomerization unit 110 via first conduit 120. In dehydrogenation-isomerization unit 110, at least a portion of the paraffins in the first hydrocarbon stream may be dehydrogenated to olefins. At least a portion of the resulting olefins and at least a portion of the olefins that were already present in the feed stream may be isomerized to produce a second hydrocarbon stream. The isomerization process converts linear olefins (e.g., unbranched olefins) into branched olefins.

The catalyst used for dehydrogenation-isomerization of the first hydrocarbon stream may be based on a zeolite catalyst modified with one or more metals or metal compounds. The catalyst used in dehydrogenation-isomerization unit 110 to treat olefins in the first hydrocarbon stream may be effective for skeletally isomerizing linear olefins in the process stream into olefins having an average number of branches per olefin molecule chain of between about 0.1 and about 2.5.

The dehydrogenation-isomerization catalyst may contain a zeolite having at least one channel with a crystallographic free channel diameter ranging from greater than about 4.2 Å and less than about 7 Å, measured at room temperature, with essentially no channel present that has a free channel diameter greater than about 7 A. The catalyst may contain at least one channel having a crystallographic free diameter at the entrance of the channel within the stated range. The catalyst may not have a diameter at the entrance of a channel that exceeds the 7 Å upper limit of the range. Zeolites possessing channel diameters greater than about 7 Å may be susceptible to undesirable aromatization, oligomerization, alkylation, coking and/or by-product formation of the olefins in the hydrocarbon stream. In some embodiments, a zeolite may not contain a channel having a free diameter along either of the x or y planes of greater than about 4.2 Å. The small channel size may prevent diffusion of the olefin into and out of the channel pore once an olefin becomes branched. Thus, the zeolite must have at least one channel with free diameters of that channel within the range of greater than about 4.2 Å and less than about 7 Å.

In an embodiment, an olefin molecule, due to its high carbon chain length, may not have to enter into a zeolite channel, diffuse through, and exit the other end of the channel. The rate of branching seen when passing the olefin across the zeolite may not correspond to the theoretical rate of branching if each olefin molecule were to pass through the channels. Most of the olefins may partially penetrate the channel for a distance effective to branch the portion of the chain within the channel and subsequently withdraw from the channel once isomerized. In an embodiment of a method to produce alkyl aromatic hydrocarbons, olefin molecules in a hydrocarbon stream may be branched at the ends of the olefin carbon backbone, and be substantially linear near the center of the molecule. For example, at least 25 percent of the carbons at the center may be unbranched.

In certain embodiments, a zeolite catalyst structure may contain a channel having free diameters within the range of greater than about 4.2 Å to less than about 7 Å along both the x and y planes in the [001] view. Zeolites with channel size greater than about 4.2 Å to less than about 7 Å may be referred to as medium or intermediate channel zeolites. Zeolites may have a 10-T member (or puckered 12-T member) ring channel structure in one view and a 9-T member or less (small pore) in another view. The zeolite may have no limit to the number of channels or to the channel orientation (parallel, non-interconnecting intersections, or interconnecting at any angle). The zeolite may have no limit to the size of the channels which are outside of the channel range of greater than about 4.2 Å to greater than about 7 Å. The outside channels may not have a free diameter in either of the x or y planes which is greater than about 7 Å. In some embodiments, zeolites with channels having a free diameter of about 4.2 Å or less in one or both of the x or y planes may be used.

Examples of zeolites include, but are not limited to, molecular sieves, ferrierite, AIPO-31, SAPO-11, SAPO-31, SAPO-41, FU-9, NU-10, NU-23, ZSM-12, ZSM-22, ZSM-23, ZSM-35, ZSM-48, ZSM-50, ZSM-57, SUZ-4A, MeAPO-11, MeAPO-31, MeAPO-41, MeAPSO-11, MeAPSO-31, and MeAPSO-41, MeAPSO-46, ELAPO-11, ELAPO-31, ELAPO-41, ELAPSO-11, ELAPSO-31, and ELAPSO-41, laumontite, cancrinite, offretite, hydrogen form of stilbite, the magnesium or calcium form ofmordenite and partheite. The isotypic structures of the zeolite frameworks, known under other names, may be considered equivalent. The framework compositions of zeolites are described in Flanigen et al., "Aluminophosphate Molecular Sieves and the Periodic Table," New Developments in Zeolite Science Technology; Kodansha Ltd., 1986.

Many natural zeolites such as ferrierite, heulandite and stilbite may feature a one-dimensional pore structure with a pore size slightly smaller in diameter than about 4.2 Å. U.S. Patent No. 4,795,623 to Evans, entitled "Time Effective Method For Preparing Ferrierite" and U.S. Patent No. 4,942,027 to Evans, entitled "Method for Preparing Ferrierite" describe converting channels in natural zeolites to larger channels.

In certain embodiments, zeolites may have a ferrierite isotypic framework structure (or homeotypic). The prominent structural features of ferrierite found by x-ray crystallography may be parallel channels in the aluminosilicate framework, which are roughly elliptical in cross-section. Examples of such zeolites having the ferrierite isotypic framework structure are described in European Patent No. 55 529 to Seddon et al., entitled "Zeolites;" U.S. Patent No. 4,578,259 to Morimoto et al., entitled "Process For Preparing A Crystalline Aluminosilicate;" European Patent No. 103 981 to Whittam, entitled "Zeolites," U.S. Patent No. 4,016,245 to Plank et al., entitled "Crystalline Zeolite And Method Of Preparing Same" and U.S. Patent No. 4,375,573 to Young et al., entitled "Selective Production And Reaction of P-Disubstituted Aromatics Over Zeolite ZSM-48."

In an embodiment, a hydrogen form of ferrierite (H-ferrierite) may be considered to be substantially one-dimensional, having parallel running channels. The elliptical channels may have free diameters of 4.2 Å times 5.4 Å along the x and y planes in the [001] view. The channels may be large enough to permit entry of a linear olefin and diffusion out of or through the channel of the methyl branched isoolefin. The channels may be small enough to retard coke formation. As used herein, "coke" refers to the product from thermal degradation of a molecule into smaller molecules. Methods for preparing various H-ferrierite are described in U.S. Patent No. 5,985,238 to Pasquale et al., entitled "Process For Preparing Ferrierite;" U.S. Patent No. 4,251,499 to Nanne et al.; entitled "Process For The Preparation Of Ferrierite;" U.S. Patent No. 4,795,623 to Evans, entitled "Time Effective Method For Preparing Ferrierite" and U.S. Patent No. 4,942,027 to Evans, entitled "Method for Preparing Ferrierite."

In certain embodiments, a dehydrogenation-isomerization catalyst may be combined with a refractory oxide that serves as a binder material. Suitable refractory oxides include, but are not limited to, natural clays (e.g., bentonite, montmorillonite, attapulgite, and kaolin), alumina, silica, silica-alumina, hydrated alumina, titania, zirconia or mixtures thereof.

The weight ratio of zeolite to binder material may range from about 10:90 to about 99.5:0.5. In some embodiments, a weight ratio may range from about 75:25 to about 99:1. In other embodiments, a weight ratio of zeolite to binder material may range from about 80:20 to about 98:2. In certain embodiments, a weight ratio of zeolite to binder material may range from about 85:15 to about 95:5 on an anhydrous basis.

In certain embodiments, a dehydrogenation-isomerization catalyst may be prepared with one or more monocarboxylic acids and/or inorganic acids. In addition to the monocarboxylic and/or inorganic acids, at least one organic acid with at least two carboxylic acid groups ("polycarboxylic acid") may be used. Monocarboxylic acids may have a substituted or unsubstituted hydrocarbyl group having 1 to 20 carbon atoms. The hydrocarbyl group may be aliphatic, cyclic or aromatic. Examples of monocarboxylic acids having 1 to 20 carbon atoms include, but are not limited to, acetic acid, formic acid, propionic acid, butyric acid, caproic acid, glycolic acid, lactic acid, hydroxylbutyric acid, hydroxycyclopentanoic acid, salicylic acid, mandelic acid, benzoic acid and fatty acids. Examples of inorganic acids include, but are not limited to, nitric acid, phosphoric acid, sulfuric acid and hydrochloric acid.

A polycarboxylic acid may, in certain embodiments, be an organic acid with two or more carboxylic acid groups attached through a carbon-carbon bond linkage to a hydrocarbon segment. The linkage may be at any portion of the hydrocarbon segment. The polycarboxylic acid may have a hydrocarbon segment ranging from 0 to 10 carbon atoms. The hydrocarbon segment may be aliphatic, cyclic or aromatic. The hydrocarbon segment may have zero carbon atoms for oxalic acid with two carboxylic acid groups attached through the carbon-carbon bond. Examples of the polycarboxylic acids include, but are not limited to, tartaric acid, citric acid, malic acid, oxalic acid, adipic acid, malonic acid, galactaric acid, 1,2-cyclopentane dicarboxylic acid, maleic acid, fumaric acid, itaconic acid, phthalic acid, terephthalic acid, phenylmalonic acid, hydroxyphthalic acid, dihydroxyfumaric acid, tricarballylic acid, benzene-1,3,5-tricarboxylic acid, isocitric acid, mucic acid and glucaric acid. The polycarboxylic acids may be any isomers of the above acids or any stereoisomers of the above acids. In an embodiment, polycarboxylic acids with at least two carboxylic acid groups and at least one hydroxyl group are used. In an embodiment, polycarboxylic acids used may be citric acid, tartaric acid and malic acid.

Metals incorporated into the dehydrogenation-isomerization catalysts may promote the oxidation of coke in the presence of oxygen at a temperature greater than about 250 °C and the dehydrogenation of paraffins. "Metal(s)," as used herein, refers to metals of a zero oxidation state and/or higher oxidation states (e.g., metal oxides).

The metals used in the dehydrogenation-isomerization catalyst may be transition and/or rare earth metals. Coke oxidation-promoting metals include, but are not limited to, Groups IB, VB, VIB, VIIB, VIII of the transition metal series of the Periodic Table and/or combinations thereof. In certain embodiments, Pd, Pt, Ni, Co, Mn, Ag, Cr and/or combinations thereof may be used in the dehydrogenation-isomerization catalyst.

The amount of the metal used in the dehydrogenation-isomerization catalyst may range from about 5 parts per million ('ppm) to about 15 percent by weight. In certain embodiments, an amount of metal may range from about 5 ppm to about 10 percent by weight. In some embodiments, an amount of metal may range from about 5 ppm to about 5 percent by weight.

Smaller amounts of metals may be incorporated into the zeolite and/or binder, when noble metals such as platinum and/or palladium are used. In certain embodiments, an amount of noble metals may range from about 5 ppm to about 2 percent by weight, basis metal, of the final catalyst. In some embodiments, an amount of noble metals may range from about 5 ppm to about 1 percent by weight, basis metal, of the final catalyst. In other embodiments, an amount of noble metal(s) may range from about 5 ppm to about 3000 ppm of a final catalyst. An amount of noble metal(s) used in a dehydrogenation-isomerization catalyst may, in certain embodiments, range from about 5 ppm to about 2000 ppm of a final catalyst. The amount of noble metal(s) sufficient to promote regeneration without deteriorating the performance of the catalyst may be in the range from about 30 ppm to about 100 ppm. Higher amounts of platinum and/or palladium (e.g., greater than about 2% by weight) may have an adverse effect on the run life, olefm isomerization activity and/or selectivity of the catalyst.

In an embodiment, zeolite powder and alumina powder may be mixed (e.g., mulling) with water and one or more compounds of the catalytic metal; and the resulting mixture may be formed into a pellet. Catalysts prepared by mulling may have superior olefin isomerization performance over catalysts prepared by impregnation. The term "mulling," as used herein, refers to mixing of powders to which sufficient water has been added to form a thick paste and wherein the mixing is accompanied by concomitant shearing of the paste. Commercially available mullers such as the Lancaster Mix Muller and the Simpson Mix Muller may be used.

The pellet may be formed, in some embodiments, by extrusion. One or more peptizing acid, (e.g., nitric acid, acetic acid, citric acid or mixtures thereof) may be added to the mixture and optional extrusion aids such as cellulose derivatives (e.g., METHOCEL®F4M, hydroxypropyl methylcellulose, manufactured by The Dow Chemical Company) may be utilized. The amounts of peptizing acid used may be determined by routine experimentation to provide a plastic, extrudable material. "Pellets," as used herein, refers to any shape or form as long as the extruded materials are consolidated.

In certain embodiments, a noble metal such as platinum and/or palladium may be added to the zeolitic catalyst after the catalyst is pelletized. Common metal incorporation methods known to those skilled in the art (e.g., impregnation, noble metal ion exchange, co-mulling) may be used to produce a working catalyst useful in dehydrogenation-isomerization of paraffins. Addition of noble metals to the catalyst may aid in the dehydrogenation reaction of paraffins. Pellets containing noble metals may be calcined at a temperature ranging from about 200 °C to about 700 °C. In certain embodiments, calcination temperatures may range from about 300°C to about 600°C. In some embodiments, calcination temperatures may range from about 450 °C to about to about 525°C.

The dehydrogenation-isomerization catalyst may be contacted with the first hydrocarbon stream in dehydrogenation-isomerization unit 110 under a variety of conditions to dehydrogenate the paraffins and isomerize the resulting olefins. In dehydrogenation-isomerization unit 110, reaction temperatures may range from about 300 °C to about 700 °C. A reaction temperature, in some embodiments, may range from about 350 °C to about 550 °C. A total pressure of dehydrogenation-isomerization unit 110 during the reaction may range from than about 0.10 atmosphere (10 kPa) to about 15.0 atmospheres (1520 kPa). In order to inhibit coking, hydrogen may be fed together with the first hydrocarbon stream. Hydrogen gas and paraffins present in the first hydrocarbon stream may be fed at a hydrogen gas to paraffin molar ratio in a range of from about 0.1 to about 20. In certain embodiments, a hydrogen gas to paraffin molar ratio may be in the range of about 1 to about 10.

Residence time in dehydrogenation-isomerization unit 110 may be selected such that a conversion level of paraffins to olefins may be kept below 40 mole percent In an embodiment, conversion level may range from 5 mole percent to 30 mole percent. By keeping conversion levels low, side reactions (e.g., diene formation and cyclization reactions) may be minimized. Conversion levels may be increased, however, by increasing the reaction temperature and/or the residence time provided side reactions remain below acceptable limits. Olefins produced in dehydrogenation-isomerization unit 110 may have a higher degree of branching than the paraffin feed to the dehydrogenation-isomerization unit. It should be understood that the concentration of olefins produced via dehydrogenation-isomerization 110 may be limited by the thermodynamic equilibrium of olefins and paraffins at a given reaction temperature.

Branched olefins produced in dehydrogenation-isomerization unit 110 may include methyl, ethyl and/or longer carbon chain branches. Hydrogen Nuclear Magnetic Resonance (¹H NMR) techniques may be used to analyze olefin compositions. Branched olefins may include quaternary and/or tertiary aliphatic carbons. In certain embodiments, an amount of quaternary and/or tertiary aliphatic carbons produced in an isomerization unit may be minimized. ¹H NMR analysis of olefins produced by the dehydrogenation-isomerization unit may indicate the extent of isomerization of the olefins in a hydrocarbons stream.

The presence of quaternary carbon atoms may be determined using carbon 13 (¹³C) NMR techniques. The type of branching (e.g., methyl, ethyl, propyl or larger groups) may be determined by hydrogenation of the olefin mixture and ¹³C NMR analysis of the hydrogenated olefins solution.

In an embodiment, an average number of branches per olefin molecule present in the branched olefin composition is between about 0.1 and about 2.5. In other embodiments, an average number of branches per olefin molecule present in the branched olefin composition is between about 0.7 and about 2.5. In certain embodiments, when the feed stream contains greater than 70 percent linear olefins, an average number of branches per olefin molecule present in a branched olefm composition is between about 0.2 and about 1.5. The degree of branching in the product may be controlled by controlling process conditions used in the dehydrogenation-isomerization unit. For example, high reaction temperatures and lower feed rates may result in a higher degree of branching. Methyl branches may represent between about 20 percent to about 99 percent of the total number of branches present in olefin molecules. In some embodiments, methyl branches may represent greater than about 50 percent of a total number of branches in olefin molecules. A number of ethyl branches in olefin molecules may represent, in certain embodiments, less than about 30 percent of the total number of branches. In other embodiments, a number of ethyl branches, if present, may be between about 0.1 percent and about 2 percent of the total number of branches. Branches other than methyl or ethyl, if present, may be less than about 10 percent of the total number of branches.

The number of aliphatic quaternary and/or tertiary carbon atoms present in the branched olefin composition may be less than about 2 percent of the carbon atoms present. In an embodiment, aliphatic quaternary and/or tertiary carbons present are less than about 1 percent of carbon atoms present. For applications in which biodegradability is important, a number of aliphatic quaternary carbon atoms may be less than about 0.5 percent of carbon atoms present. In an embodiment, a number of aliphatic quaternary and/or tertiary carbon atoms is less than about 0.3 percent of carbon atoms present. In other embodiments, a number of aliphatic quaternary carbon atoms present in the branched olefin composition is between about 0.01 percent and about 0.3 percent of the aliphatic carbon atoms present.

The second hydrocarbon stream may exit dehydrogenation-isomerization unit 110 and be transferred to other processing units (e.g., alkylation) via second conduit 130. At least a portion of the second hydrocarbon stream may exit dehydrogenation-isomerization unit 110 and be transported to alkylation unit 140 via second conduit 130, as depicted in System 100 of FIG. 1.

Alkylation of aromatic hydrocarbons by at least a portion of branched olefins produced in dehydrogenation-isomerization unit 110 may be conducted using various types of reactors. In certain embodiments, a process may be carried out in a batch wise fashion by adding the catalyst and aromatic hydrocarbons to a reactor, heating the mixture to a reaction temperature, and then adding the olefinic or aromatic hydrocarbons to the heated mixture.

Alkylation unit 140 may have several points of entry for additional process streams. As used herein, "stream entering into the alkylation unit" refers to the entry of process streams into the alkylation unit through one or more entry points. Examples of such process streams include, but are not limited to, streams from dehydrogenation-isomerization unit 110, a diluent hydrocarbon stream, gases and/or other hydrocarbon streams that include olefins and paraffins derived from other processes.

In an embodiment, at least a portion of olefins in the second hydrocarbon stream, produced by dehydrogenation-isomerization unit 110, is contacted with aromatic hydrocarbons (e.g., benzene) using a variety of alkylating conditions. At least a portion of the resulting alkyl aromatic hydrocarbons are monoalkylated aromatic hydrocarbons having a branched alkyl group. Minimization of other alkylation products, such as dialkylation and higher alkylation products, may be controlled by process conditions (e.g., molar ratio, reaction temperatures, catalyst type and reactant concentrations) in alkylation unit 140.

In an embodiment, paraffins and other non-reactive components in the second hydrocarbon stream act as a diluent in the alkylation step. The presence of diluents in alkylation unit 140 may help control the temperature of the alkylation reaction. Diluents in alkylation unit 140 may also improve the selectivity of the alkylation process. Increased reaction temperatures in alkylation unit 140 may result in the formation of by-products (e.g., dialkylation and higher alkylation products).

The stoichiometry of the alkylation reaction requires only one mole of aromatic hydrocarbon compound per mole of total mono-olefins. Using 1:1 molar conditions, however, tends to produce mixtures of olefin oligomers and/or polymers, monoalkyl aromatic hydrocarbons, dialkyl-, trialkyl- and possibly highly polyalkylated aromatic hydrocarbons or unreacted olefins. A molar ratio as close to 1:1 as possible may maximize utilization of the aromatic hydrocarbon and minimize recycle of unreacted aromatic hydrocarbons or unreacted olefins. The molar proportion of aromatic hydrocarbon to total mono-olefin may influence both conversion and selectivity of the alkylation reaction. In certain embodiments, a molar ratio of total aromatic hydrocarbon per mole of total mono-olefins may be set between about 3:1 and about 100:1 to maximize formation of monoalkyl aromatic hydrocarbons. In some embodiments, an aromatic hydrocarbon to olefin ratio may be from about 5:1 to about 50:1. In other embodiments, an aromatic hydrocarbon to olefin ratio may be from about 5:1 to about 35:1. In certain embodiments, an aromatic hydrocarbon to total olefin ratio may be from about 8:1 to about 30:1.

In an embodiment, alkylation conditions for alkylation of aromatic hydrocarbons in alkylation unit 140 may include the use of a Friedel-Crafts catalyst type. The Friedel-Crafts catalyst may be an acidic inorganic material. Examples of acidic inorganic materials include, but are not limited to, mineral acids such as sulfuric acid containing less than about 10 percent water, hydrofluoric acid containing less than about 10 percent water, liquefied anhydrous hydrogen fluoride, and/or other inorganic materials used in combination with hydrofluoric acid. Other inorganic materials may include, but are not limited to, Lewis acids such as anhydrous aluminum chloride, anhydrous aluminum bromide and boron trifluoride.

In certain embodiments, an alkylation catalyst may be a molecular sieve such as ITQ-21 in the acidic form. The synthesis and structures of molecular sieve catalysts are described by Corma, et al. in "A large-cavity zeolite with wide pore windows and potential as an oil refining catalyst," *Nature,* 2002, 418:514.

In certain embodiments, a catalyst used in alkylation unit 140 is based on a zeolite catalyst that may or may not be modified with a metal or metal compound. Zeolite alkylation catalysts are described in U.S. Patent Application Serial No. 10/075,318 entitled "A Process For Preparing (Branched-Alkyl) Arylsulfonates and (Branched-Alkyl) Arylsulfonate Composition," U. S. Patent No. 6,111,158 to Marinangeli et al. entitled, "Process For Producing Arylalkanes At Alkylation Conditions Using A Zeolite Having a NES Zeolite Structure Type" and U.S. Patent No. 5,041,402 to Schoennagel et al., entitled, "Thermally Stable Noble Metal-Containing Zeolite Catalyst." The zeolite catalyst may have at least one channel with a crystallographic free channel diameter ranging from greater than about 4 Å to less than about 9 Å with the understanding that when the pores have an elliptical shape, the larger pore size dimension is the dimension to be considered. In an embodiment, a pore size dimensions may range between about 5.5 Å to about 7 Å. The pore size dimensions of zeolites are described in W. M. Meier et al., "Atlas of Zeolite Structure Types," Fourth revised edition, 1996, Elsevier.

In alkylation unit 140, at least a portion of the olefins in the second hydrocarbon stream and at least a portion of the aromatic hydrocarbons may be reacted under alkylation conditions in the presence of the alkylation catalyst. Reaction temperatures for the alkylation reaction may range between greater than about 30 °C and less than about 300 °C. In certain embodiments, reaction temperatures may range between greater than about 100 °C and less than about 250 °C. The alkylation reaction may be conducted in at least a partial liquid phase, in an all-liquid phase or at supercritical conditions. In certain embodiments, pressures in the alkylation unit are sufficient to maintain reactants in the liquid phase. Pressures used in alkylation unit 140 may depend upon the olefin type, the aromatic hydrocarbons type and/or the reaction temperature. Pressures in alkylation unit 140 may range between about 3 atmospheres and about 70 atmospheres (304 kPa-7095 kPa). In certain embodiments, pressures may range between about 20 atmospheres and about 35 atmospheres (2025-3545 kPa).

Alkylation conditions in alkylation unit 140 may be maintained to minimize skeletal isomerization of the branched olefins. Alkylation conditions may also be maintained to produce alkyl aromatic hydrocarbons with an alkyl group that corresponds to the branching of the olefins produced in dehydrogenation-isomerization unit 110. "Skeletal isomerization during alkylation," as used herein, refers to isomerization during alkylation that changes the branching position of an olefin or alkyl aromatic hydrocarbon product. Accordingly, alkylation conditions may be set such that a number of quaternary aliphatic carbon atoms in the olefin and the alkyl aromatic hydrocarbon product may remain unchanged during the alkylation reaction.

A general class of branched alkyl aromatic compounds produced in alkylation unit 140 may be characterized by the chemical formula R-Y, in which Y represents an aromatic hydrocarbyl radical (e.g., a phenyl radical) and R represents a radical derived from an olefin produced in dehydrogenation-isomerization unit 110. An olefin may have a carbon number in the range from 7 to 16. In certain embodiments, an olefin carbon number may range from 10 to 16. An olefin carbon number may, in other embodiments, range from 10 to 13. R may be a branched alkyl radical. Branches on a branched alkyl radical may include, but are not limited to, methyl, ethyl and/or longer carbon chains. An average number of branches per alkyl molecule present in the alkyl composition may be equal to a number of branches in the olefin produced in dehydrogenation-isomerization unit 110 (e.g., between 0.1 and 2.0).

In certain embodiments, additional hydrocarbon streams may be used to control reaction conditions and/or optimize the concentration of paraffins and unreacted olefins in dehydrogenation-isomerization unit 110 and/or alkylation unit 140. In certain embodiments, it may be desirable to adjust the olefin and paraffin concentration depending on the source of the olefin stream. A paraffinic stream may be added to a process stream that contains greater than about 50 percent mono-olefins upstream of an alkylation unit to produce a process stream that is less than about 50 percent mono-olefins. In some embodiments, a hydrocarbon stream containing about 20 percent linear olefins and 80 percent paraffins may be added to a process stream containing primarily branched olefins upstream of an alkylation unit to produce a combined stream. Alkylation of aromatic hydrocarbons with the combined stream may result in a mixed stream containing branched and linear alkyl aromatic hydrocarbons.

In an embodiment, a third hydrocarbon stream is introduced directly into alkylation unit 140 and/or dehydrogenation-isomerization unit 110 through one or more points of entry. In other embodiments, a third hydrocarbon stream may be combined with a process stream upstream of a alkylation unit and/or a dehydrogenation-isomerization unit. At least a portion of a third hydrocarbon stream may be introduced into second conduit 130 via third conduit 150 upstream of alkylation unit 140 to produce a combined stream. In other embodiments, an olefin content of the third hydrocarbon stream ranges between about 1 percent and about 99 percent relative to the total hydrocarbon content. In certain embodiments, an olefin content of the third hydrocarbon stream may be between about 5 percent and about 15 percent relative to the total hydrocarbon content. In other embodiment, an olefin content of the third hydrocarbon stream may be greater than 80 percent relative to the total hydrocarbon content.

In an embodiment, an olefin content of a third hydrocarbon stream ranges between about 1 percent and about 99 percent relative to the total hydrocarbon content. In certain embodiments, an olefin content of a third hydrocarbon stream may be between about 5 percent and about 15 percent relative to the total hydrocarbon content. In other embodiments, an olefin concentration of the third hydrocarbon stream may be greater than about 80 percent by weight.

In some embodiments, the third hydrocarbon stream may include linear olefins. The addition of a stream that includes linear olefins downstream from the dehydrogenation-isomerization unit allows the creation of an alkylation feed stream that includes a mixture of linear and branched olefins. By introducing a stream including branched and linear olefins into alkylation unit 140, a mixture of branched and linear alkyl aromatic products may be obtained. Varying the amount of linear olefins added to the alkylation feed stream may control the ratio of linear to branched alkyl aromatic products. A mixture of branched and linear alkyl aromatic hydrocarbons may have improved properties when converted to alkyl aromatic surfactants. Examples of improved properties include, but are not limited to, low skin and eye irritation, foaming properties, biodegradability, cold-water solubility and cold-water detergency. Applications for these surfactants include, but are not limited to, personal care products, household and industrial laundry products, hand dishwashing products, machine lubricant additives and lubricating oil formulations.

The alkylation reaction mixture stream may enter separator 160 via fourth conduit 170. In separator 160 at least two streams, an unreacted hydrocarbons stream and an alkyl aromatic hydrocarbon stream may be produced. The unreacted hydrocarbons stream may be separated into an aromatic hydrocarbons stream and a paraffins and unreacted olefins stream using techniques known in the art (e.g., distillation, solid/liquid separation, absorption, solvent extraction, etc.). In some embodiments, paraffins, unreacted olefins, alkyl aromatic hydrocarbons and reaction by-products may be produced as one stream and separated by techniques known in the art. The separated aromatic hydrocarbons may be transferred to alkylation unit 140 via fifth conduit 180. At least a portion of the unreacted olefin and paraffins stream may be combined with other process streams, sent to other processing units and/or stored on site. The alkyl aromatic hydrocarbons product stream may be transported via sixth conduit 190 to be stored on site, sold commercially, transported off-site and/or utilized in other processing units.

At least a portion of the paraffins and unreacted olefins stream may be combined via seventh conduit 195 with the first hydrocarbon stream in first conduit 120 to produce a combined stream. The combined stream may be introduced into dehydrogenation-isomerization unit 110 via first conduit 120 and at least a portion of the olefins in the combined stream may be isomerized to branched olefins. In some embodiments, at least a portion of the paraffins and unreacted olefins stream is introduced directly into dehydrogenation-isomerization unit 110 via one or more points of entry. Because the paraffins and unreacted olefins may be recycled to dehydrogenation-isomerization unit 110 as one stream, the process may be more efficient, resulting in a higher overall throughput. The higher throughput will increase overall yield of alkyl aromatic hydrocarbons.

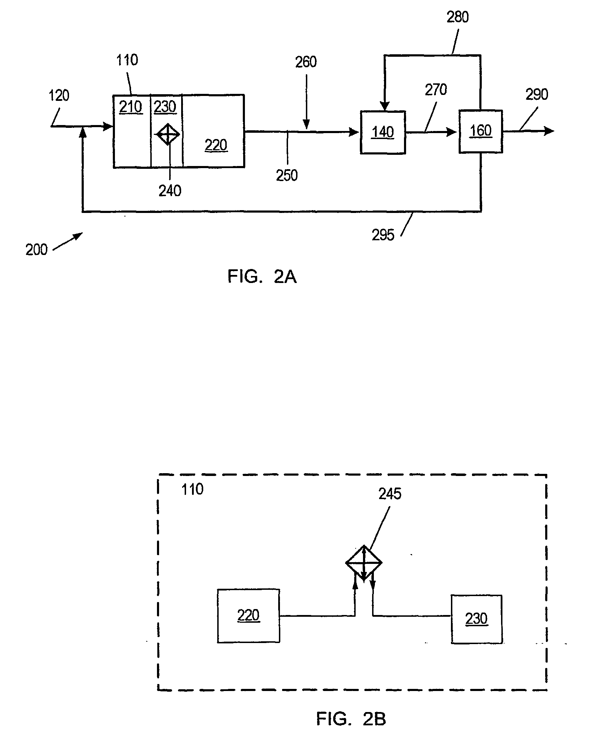

In certain embodiments, dehydrogenation-isomerization unit 110 may be separated into a plurality of zones to control reaction temperatures and/or prevent unwanted side reactions (e.g., diene formation and/or cyclization reactions). Referring to System 200 depicted in FIG. 2A, a first hydrocarbon stream containing g paraffins and unreacted olefins may be introduced into dehydrogenation-isomerization unit 110 via first conduit 120. The composition of the first hydrocarbon stream may be the same as previously described for System 100. Dehydrogenation-isomerization unit 110 may be divided into a plurality of zones. The plurality of zones may include, but is not limited to, a first reaction zone, a transition zone and a second reaction zone. In first reaction zone 210, at least a portion of the paraffins in the first hydrocarbon stream may be dehydrogenated to olefins to produce an olefinic stream. The process stream may then pass through transition zone 230 into second reaction zone 220. In second reaction zone 220 at least a portion of the olefins in the process stream may be isomerized to branched olefins to produce a second hydrocarbon stream.

The dehydrogenation catalyst in first reaction zone 210 may be selected from a variety of catalyst types. At least a portion of the unreacted paraffins in the hydrocarbon stream may be dehydrogenated to produce an olefinic hydrocarbon stream by use of a catalyst selected from a wide range of catalyst types. For example, the catalyst may be include a metal or metal compound deposited on a porous support. The metal or metal compound may be selected from, but is not limited to, chrome oxide, iron oxide and/or noble metals. "Noble metals" as used herein refers to the metals of the group that includes platinum, palladium, iridium, ruthenium, osmium and rhodium.

Techniques of preparing catalysts, for performing the dehydrogenation step and for performing associated separation steps are known in the art. For example, suitable procedures for preparing catalysts and performing the dehydrogenation step are described in U.S. Patent No. 5,012,021 to Vora et al., entitled, "Process For the Production of Alkyl Aromatic Hydrocarbons Using Solid Catalysts;" U.S. Patent No. 3,274,287 to Moore et al., entitled, "Hydrocarbon Conversion Process and Catalyst;" U.S. Patent No. 3,315,007 to Abell et al., entitled, "Dehydrogenation of Saturated Hydrocarbons Over Noble-Metal Catalyst;" U.S. Patent No. 3,315,008 to Abell et al., entitled, "Dehydrogenation of Saturated Hydrocarbons Over Noble-Metal Catalyst;" U.S. Patent No. 3,745,112 to Rausch, entitled, "Platinum-Tin Uniformly Dispersed Hydrocarbon Conversion Catalyst and Process;" U.S. Patent No. 4,506,032 to Imai et al., entitled, "Dehydrogenation Catalyst Composition" and U.S. Patent No. 4,430,517 to Imai et al., entitled, "Dehydrogenation Process Using a Catalytic Composition."

Reaction temperatures in first reaction zone 210 may range from about 300 °C to about 600 °C. In some embodiments, reaction temperatures may range from about 450 °C to about 550 °C. The total pressure in the dehydrogenation zone may range from about 0.10 atmosphere (10 kPa) to about 15.0 atmospheres (1520 kPa). In order to prevent coking, hydrogen may be fed together with the unreacted first hydrocarbon stream. Hydrogen and paraffins present in the unreacted first hydrocarbon steam may be fed at a hydrogen to paraffin molar ratio in the range of from about 0.1 to about 20. In an embodiment, a hydrogen to paraffin molar ratio may be in the range of about 1 to about 10.

The residence time in first reaction zone 210 may be selected such that conversion level of the paraffins to olefins may is below about 50 mole percent. In certain embodiments, a conversion level of the paraffins to olefins may be kept in the range of from about 10 mole percent to about 20 mole percent. By keeping the conversion level low, side reactions (e.g., diene formation and cyclization reactions) may be prevented. The olefinic hydrocarbon stream may exit first reaction zone 210, pass through transition zone 230 and enter second reaction zone 220. Transition zone 230 may include heat exchanger 240. Heat exchanger 240 may reduce the temperature of the olefinic hydrocarbon stream. In an embodiment, first reaction zone 210 and second reaction zone 220 in dehydrogenation-isomerization unit 110 may be separate units, as depicted in FIG. 2B, with heat exchanger 240 positioned between the two units.

After the olefinic hydrocarbon stream enters second reaction zone 220, at least a portion of the olefins are isomerized to branched olefins to produce a second hydrocarbon stream. The composition and level of branching of the second hydrocarbon stream may be analyzed by ¹H NMR techniques. In an embodiment, the olefmic stream may pass from first reaction zone 210 directly to second reaction zone 220. At least a portion of the olefins in the olefinic stream are isomerized to branched olefins in second reaction zone 220.

A catalyst used for isomerization of the olefins to branched olefins in second reaction zone 220 may be the same as that described in U.S. Patent No. 5,510,306 to Murray, entitled "Process For Isomerizing Linear Olefins to Isoolefins." Temperatures in second reaction zone 220 may range from about 200 ° C to about 500°C. In some embodiments, reaction temperatures in the first reaction zone and the second reaction zone are substantially the same. In such embodiments, the use of a heat exchanger is not required. Typically, however, the reaction temperature of the second reaction zone is less than the reaction temperature of the first reaction zone. The heat exchanger may lower the temperature of the stream leaving the first reaction zone to the appropriate temperature for reaction in the second reaction zone. Pressure maintained in second reaction zone 220 may be at a hydrocarbon partial pressure ranging from 0.1 atmosphere (10.1 kPa) to 10 atmospheres (1011 kPa). In an embodiment, a partial pressure may range from above about 0.5 (510 kPa) atmosphere to about 2 atmospheres (203 kPa).

The second hydrocarbon stream may exit second reaction zone 220 via second conduit 250 and enter alkylation unit 140. At least a portion of a third hydrocarbon stream may be introduced into second conduit 250 via third conduit 260, as depicted in FIG. 2A to form a combined stream. The combined stream may be introduced into alkylation unit 140 via second conduit 250. At least a portion of the olefins in the combined stream may alkylate at least a portion of the aromatic hydrocarbons to produce alkyl aromatic hydrocarbons. In an embodiment, a third hydrocarbon stream may be introduced directly into alkylation unit 140 through one or more points of entry as previously described for System 100 in FIG. 1.

Separation of at least a portion of unreacted olefins and at least a portion of paraffins from the alkyl aromatic hydrocarbons may be performed as previously described for System 100 in FIG. 1. The alkylation reaction mixture stream may enter separator 160 via fourth conduit 270. At least two streams, an unreacted hydrocarbons stream and an alkyl aromatic hydrocarbons stream, may be produced in separator 160. At least a portion of the separated aromatic hydrocarbons may be transferred to alkylation unit 140 via fifth conduit 280. At least a portion of the paraffins and unreacted olefins stream may be combined with other process streams, sent to other processing units and/or stored on site. The alkyl aromatic hydrocarbons product stream may be transported via sixth conduit 290 to be stored on site, sold commercially, transported off-site and/or utilized in other processing units.

At least a portion of the paraffins and unreacted olefins stream may be combined via seventh conduit 295 with the first hydrocarbon stream in first conduit 120 to produce a combined hydrocarbon stream. The combined hydrocarbon stream may enter first reaction zone 210 of dehydrogenation-isomerization unit 110 via first conduit 120. The combined hydrocarbon stream entering first reaction zone 210 continues the dehydrogenation-isomerization process and alkylation process to produce alkyl aromatic hydrocarbons. By recycling the paraffins and unreacted olefins stream, yield of product may be maximized. In an embodiment, a paraffins and unreacted olefins stream may directly enter dehydrogenation-isomerization unit 110 through one or more points of entry.

In an embodiment, a catalyst in dehydrogenation-isomerization unit 110 may be used in a stacked bed configuration. A stacked bed configuration may allow for the use of one or more catalyst in the reactor. A catalyst for dehydrogenation of paraffins and a catalyst for isomerization of olefins may enhance the selectivity of the catalysts and/or the process. System 300 in FIG. 3, depicts a stacked bed configuration of dehydrogenation-isomerization unit 110. The operating conditions of the stacked bed configuration may be the same as for two-zone system described for System 200. The first hydrocarbon stream may enter the dehydrogenation zone 210 via first conduit 310. The dehydrogenation catalyst used in the stacked bed configuration may have the properties as described in U.S. Patent No. 4,506,032 to Imai et al., entitled "Dehydrogenation Catalyst Composition."

The process stream may pass into isomerization zone 220. In certain embodiments, a temperature decrease from dehydrogenation zone 210 to isomerization zone 220 may be necessary to prevent cracking of the first hydrocarbon stream as it enters the isomerization zone. Cool hydrogen gas may be introduced to dehydrogenation zone 210 via gas conduit 320 to control temperatures in dehydrogenation zone 210. In isomerization zone 220, at least a portion of the olefins in the process stream may be isomerized to branched olefins to produce a second hydrocarbon stream.

In certain embodiments, an isomerization catalyst may be the same as in U. S. Patent No. 5,648,584 to Murray, entitled "Process for Isomerizing Linear Olefins to Isoolefins" and U.S. Patent No. 5,648,585 to Murray et al., entitled "Process for Isomerizing Linear Olefins to Isoolefins," which describe catalysts and process conditions to skeletally isomerize linear olefins to branched olefins.

In an embodiment, linear olefins in a first *hydrocarbon stream are isomerized in isomerization* zone 220 by contacting at least a portion of the first hydrocarbon stream with a zeolite catalyst. The zeolite catalyst may have at least one channel with a crystallographic free channel diameter ranging from greater than 4.2 Å and less than about 7 Å. The zeolite catalyst may have an elliptical pore size large enough to permit entry of a linear olefin and diffusion, at least partially, of a branched olefin. The pore size of the zeolite catalyst may also be small enough to retard coke formation.

In some embodiments, about 0.01 weight percent to about 5 weight percent of a noble metal may be added to an isomerization catalyst used in a stacked bed configuration to increase the dehydrogenation activity of the zeolite catalyst. When a noble metal such as platinum and/or palladium is added to the zeolite catalyst, common metal incorporation methods known to those skilled in the art, such as impregnation, noble metal ion exchange and co-mulling may be used to produce a working catalyst useful in dehydrogenation-isomerization of paraffins.

The second hydrocarbon stream may exit second reaction zone 220 and enter alkylation unit 140 via second conduit 330. At least a portion of a third hydrocarbon stream may be introduced into second conduit 330 via third conduit 340, as depicted in FIG. 3. The combined stream may be introduced into alkylation unit 140 via second conduit 330. At least a portion of the olefins in the combined stream may alkylate at least a portion of the aromatic hydrocarbons to produce alkyl aromatic hydrocarbons. The composition of the third hydrocarbon stream may be the same as previously described for System 100.

Separation of at least a portion of unreacted olefins and at least a portion of paraffins from the alkyl aromatic hydrocarbons may be performed as previously described for System 100. The alkylation reaction mixture stream may enter separator 160 via fourth conduit 350. Separation of the alkylation reaction mixture may be the same as described for System 100. The separated aromatic hydrocarbon may be transferred to alkylation unit 140 via fifth conduit 360. At least a portion of the paraffins and unreacted olefins stream may be combined with other process streams, sent to other processing units and/or stored on site via sixth conduit 370. The alkyl aromatic hydrocarbon product stream may be transported via seventh conduit 380 to be stored on site, sold commercially, transported off-site and/or utilized in other processing units.

At least a portion of the paraffins and unreacted olefins stream may be introduced into first conduit 310 via sixth conduit 370 to produce a combined stream. The combined stream may be introduced into dehydrogenation-isomerization unit 110 via first conduit 310. At least a portion of the paraffins in the combined stream may be dehydrogenated and isomerized to branched olefins. In an embodiment, at least a portion of a paraffins and unreacted olefins stream may be introduced directly into dehydrogenation-isomerization unit 110 via one or more points of entry. In certain embodiments, one or more combined process streams and at least a portion of an paraffins and unreacted olefins stream may be introduced directly into dehydrogenation-isomerization unit 110 through one or more points of entry.

The alkyl aromatic hydrocarbon product stream produced from Systems 100, 200, and/or 300 may be sulfonated in a sulfonation unit to form alkyl aromatic sulfonates. In certain embodiments, the alkyl aromatic hydrocarbons may contain branched alkyl groups. Sulfonation of at least a portion of the aromatic hydrocarbons in the alkyl aromatic hydrocarbon product stream may be performed by any method of sulfonation known in the art. Examples of such methods include sulfonation using sulfuric acid, chlorosulfonic acid, oleum or sulfur trioxide. Details of a sulfonation method involving an air/sulfur trioxide mixture are described in U. S. Patent No. 3,427,342 to Brooks et al., entitled "Continuous Sulfonation Process." In an embodiment, a sulfur trioxide to alkyl aromatic product molar ratio used for sulfonation is 1.03.

After the sulfonation reaction is complete, the sulfonation reaction mixture may be aged for about thirty minutes and then hydrolyzed with approximately 1 % water. The resulting acid mixture may be neutralized with a base to produce a salt of the branched alkyl aromatic hydrocarbon sulfonate. Suitable neutralization bases may include hydroxides of alkali metals and alkaline earth metals, and ammonium hydroxides, which provide a cation of a sulfonate.

The general class of branched alkyl aromatic hydrocarbon sulfonates may be characterized by the chemical formula (R-A'-SO₃)*ₙ*M. R represents a radical derived from the branched olefins, having an average carbon number in the range from 4 to 16. In an embodiment, an average carbon number ranges from 7 to 16. In another embodiment, an average carbon number ranges from 10 to 13. A' may represent a divalent aromatic hydrocarbyl radical, (e.g. a phenyl radical). M may be a cation selected from an alkali metal ion, an alkaline earth metal ion, an ammonium ion, and/or mixtures thereof and n may be a number depending on the valence of the cation(s) M, such that the total electrical charge of the complex is zero. In an embodiment, M may be sodium, magnesium or potassium ions. Magnesium and potassium ions may promote water solubility and overall performance of the alkyl aromatic hydrocarbon sulfonate. Examples of ammonium ions may include, but are not limited to, monoethanol amine, diethanol amine and triethanol amine. In certain embodiments, an ammonium ion may be represented by NH₄⁺. The resulting alkyl aromatic hydrocarbon sulfonate salt may be stored and/or sold as a solution (e.g. 30% aqueous solution), slurry (e.g., 60% aqueous slurry) and/or flakes (e.g., 90% dried flakes).

The branched alkyl aromatic sulfonates produced in the above-described processes may be used in a wide variety of applications. An application includes detergent formulations. Detergent formulations include, but are not limited to, granular laundry detergent formulations, liquid laundry detergent formulations, liquid dishwashing detergent formulations and miscellaneous formulations. Examples of miscellaneous formulations include, but are not limited to, general purpose cleaning agents, liquid soaps, shampoos and liquid scouring agents.

### EXAMPLES

**Example 1: Isomerization of Olefins in a Fischer-Tropsch derived Hydrocarbon Stream: Carbon monoxide** and hydrogen were reacted under Fischer-Tropsch process conditions to yield a hydrocarbon mixture of linear paraffins, linear olefins, a minor amount of dienes and a minor amount of oxygenates. The Fischer-Tropsch hydrocarbon stream was separated into different hydrocarbon streams using fractional distillation techniques. A hydrocarbon stream containing olefins and paraffins with an average number of carbon atoms between 8 and 10 was obtained. The composition of the resulting C₈-C₁₀ hydrocarbon stream, tabulated in Table 1, was analysed by gas chromatography.

**Table 1**

| **Fischer-Tropsch Hydrocarbon** | **Wt%** |
|---|---|
| **Stream Composition** | |
| C₇ and lighter hydrocarbons | 0.12 |
| C₈ branched olefins | 0.02 |
| C₈ linear olefins | 0.75 |
| 1-Octene | 0.69 |
| n-Octane | 2.21 |
| C₉ branched olefins | 0.16 |
| C₉ linear olefins | 8.52 |
| 1-Nonene | 8.07 |
| n-Nonane | 20.03 |
| C₁₀ branched olefins | 0.28 |
| C₁₀ linear olefins | 22.92 |
| 1-Decene | 20.87 |
| n-Decane | 41.12 |
| C₁₁ and heavier hydrocarbons | 0.21 |
| C₉-C₁₁ alcohols | 3.56 |

A zeolite catalyst used for isomerization of linear olefins in the hydrocarbon stream was prepared in the following manner. Ammonium-ferrierite (645 grams) exhibiting a 5.4% loss on ignition and exhibiting the following properties: molar silica to alumina ratio of 62:1, surface area of 369 square meters per gram (P/Po = 0.03), soda content of 480 ppm and n-hexane sorption capacity of 7.3 g per 100 g of ammonium-ferrierite was loaded into a Lancaster mix muller. CATAPAL® D alumina (91 grams) exhibiting a loss on ignition of 25.7% was added to the muller. During a five-minute mulling period, 152 milliliters of deionized water was added to the alumina/ammonium-ferrierite mixture. Next, a mixture of 6.8 grams glacial acetic acid, 7.0 grams of citric acid and 152 milliliters of deionized water was slowly added to the alumina/ammonium-ferrierite mixture in the muller to peptize the alumina. The resulting alumina/ammonium-ferrierite/acid mixture was mulled for 10 minutes. Over a period of 15 minutes, a mixture of 0.20 grams of tetraamine palladium nitrate in 153 grams of deionized water was slowly added to mulled alumina/ammonium-ferrierite/acid mixture. The resulting mixture exhibited a 90:10 ratio of zeolite to alumina and a loss on ignition of 43:5%. The zeolite/alumina mixture was shaped by extruding the mixture through a stainless steel die plate (1/16" holes) of a 2.25 inch Bonnot extruder.

The moist zeolite/alumina extrudate was dried at 125 °C for 16 hours. After drying, the zeolite/alumina extrudate was longsbroken manually. The zeolite/alumina extrudate was calcined in flowing air at 200 °C for two hours. The temperature was raised to a maximum temperature of 500 °C and the zeolite/alumina extrudate was calcined for an additional two hours to yield an isomerization catalyst. The isomerization catalyst was allowed to cool in a dessicator under a nitrogen atmosphere.

Stainless steel tubing, 1 inch OD, 0.6 inch ID and 26 inches long, was used as an isomerization reactor. A thermowell extended 20 inches from the top of the stainless steel reactor tube. To load the reactor tube, the reactor tube was inverted and a piece of glass wool was transferred down the wall of the reactor tube, over the thermowell and positioned at the bottom of the reactor tube to serve as a plug for the reactor tube. Silicon carbide (20 mesh) was added to a depth of about 6 inches to the reactor tube. A second piece of glass wool was placed over the silicon carbide. A mixture of 6.0 grams of the isomerization catalyst particles (6-20 mesh) and 45 grams of fresh silicon carbide (60-80 mesh) was added to the reactor tube in two parts. The two-part addition distributed the isomerization catalyst evenly in the reactor tube and resulted in an isomerization catalyst bed of about 10 inches in length. A third piece of glass wool was added to the top of the catalyst in the reactor tube. Silicon carbide (20 mesh) was layered onto the third piece of glass wool. A fourth piece of glass wool was positioned over the silicon carbide to serve as a plug for the bottom of the reactor tube. To monitor the temperature of the reaction at various points in the reactor tube, a multipoint thermocouple was inserted into the thermowell of the reactor tube. The temperature above, below and at three different places in the catalyst bed was monitored. The reactor tube was inverted and installed in the furnace. The reactor tube was heated to an operating temperature of 280 °C over a four-hour period under flowing nitrogen. Once the temperature of 280 °C was obtained, the reactor tube was held at the operating temperature for an additional two hours to condition the isomerization catalyst.

After conditioning the isomerization catalyst, the hydrocarbon stream was pumped through the reactor tube at a flow rate of 60 g/hr. Nitrogen, at a flow rate of 6 L/hr, was passed over the isomerization catalyst simultaneously with the hydrocarbon stream. The hydrocarbon stream was vaporized before contacting the isomerization catalyst. The reactor tube was operated at an outlet pressure of 20 kPa above atmospheric pressure.

In Table 2, the weight percent of C₈-C₁₀ branched olefins, C₈-C₁₀ linear olefins and C₈-C₁₀ paraffins in the hydrocarbon stream at 0 hours and in the reactor tube effluent after 24 and 48 hours of isomerization is tabulated. Greater than 90% of the linear olefins in the hydrocarbon stream were converted into branched olefins in the isomerization reactor. During the isomerization step, a small amount of material boiling below C₈ was generated from cracking side reactions. In addition, a portion of the C₉C₁₁ alcohols present in the feed was dehydrated to yield additional olefins in the product. The average number of alkyl branches on the C₈-C₁₀ olefins in the product was found to be 1.0 as determined by the ¹H NMR techniques.

**Table 2**

| **Fischer-Tropsch Hydrocarbon** | **0 Hr** | **24 Hr** | **48 Hr** |
|---|---|---|---|
| **Stream Composition During Isomerization** | **Wt%** | **Wt%** | **Wt%** |
| **Reaction** | | | |
| C₈-C₁₀ branched olefins | 0.46 | 33.04 | 33.16 |
| C₈-C₁₀ linear olefins | 32.19 | 2.52 | 2.54 |
| C₈-C₁₀ paraffins | 63.19 | 63.32 | 63.27 |
| Branched to linear C₈₋₁₀ olefin ratio | 0.1 | 13.1 | 13.1 |

**Example 2. Isomerization of 1-Dodeceme:** 1-Dodecene was obtained from Shell Chemical Co. The composition of 1-dodecene, as assayed by gas chromatography, is tabulated in Table 3.

**Table 3**

| **1-Dodecene Composition** | **Wt %** |
|---|---|
| 1-Dodecene | 98.0 |
| Other C₁₀-C₁₄ olefins | 1.2 |
| <C₁₀ hydrocarbons | 0.2 |
| >C₁₄ hydrocarbons | 0.2 |
| Paraffins | 0.4 |
| Total C₁₀-C₁₄ hydrocarbons | 99.6 |

1-Dodecene was isomerized using the same reactor tube design and isomerization catalyst preparation as described in Example 1. A stream of 1-dodecene was pumped through a reactor tube at a flow rate of 90 g/hr. Nitrogen, at a flow rate of 6 L/hr, was passed over the isomerization catalyst simultaneously with the stream of 1-dodecene. The stream of 1-dodecene was vaporised before contacting the isomerization catalyst. The reactor tube was operated at an outlet pressure of 20 kPa above atmospheric pressure and a temperature of 290°C.

Table 4 is a tabulation of the weight percent of hydrocarbons with carbon numbers less than C₁₀, from C₁₀-C₁₄ and greater than C₁₄ molecules in 1-dodecene at 0 hours and the reactor tube effluent after 168 and 849 hours. Linear C₁₀-C₁₄ olefins were converted in a 94% yield to branched C₁₀-C₁₄ olefins after a 168 hr processing time. During the isomerization step less than 3 weight percent of material boiling below C₁₀ was generated from cracking side reactions. The average number of alkyl branches on the C₁₀-C₁₄ olefins in the product was determined to be 1.3 by the ¹H NMR techniques.

**Table 4**

| **1-Dodecene Stream Composition During** | **0 Hr** | **168 Hr Wt%** | **849 Hr** |
|---|---|---|---|
| **Isomerization Reaction** | **Wt%** | | **Wt%** |
| <C₁₀ hydrocarbons | 0.2 | 2.5 | 2.4 |
| C₁₀-C₁₄ hydrocarbons | 99.6 | 97.2 | 97.4 |
| >C₁₄ hydrocarbons | 0.2 | 0.3 | 0.2 |
| Branched C₁₀-C₁₄ olefins | 0.6 | 93.2 | 93.4 |
| Linear C₁₀-C₁₄ olefins | 99.0 | 2.8 | 2.9 |
| Paraffins | 1.0 | 2.0 | 1.9 |

**EXAMPLE 3. DEHYDROGENATION OF DODECANE WITH MINIMAL ISOMERIZATION:** Dodecane was obtained from Aldrich Chemical Company and stored under nitrogen before being processed. The composition of dodecane, as assayed by gas chromatography, is tabulated in Table 5.

**Table 5**

| **Dodecane Composition** | **Wt%** |
|---|---|
| Dodecane | 99.3 |
| <C₁₀ hydrocarbons | <0.1 |
| C₁₀, C₁₁, C₁₃ and C₁₄ hydrocarbons | <0.6 |
| >C₁₄ hydrocarbons | <0.1 |
| Other C₁₀-C₁₄ Olefins | <0.1 |

A paraffin dehydrogenation catalyst was prepared according to Example 1 (catalyst A) of U.S. Patent No. 4,430,517 to Imai et al., entitled "Dehydrogenation Process Using A Catalytic Composition." The resulting catalyst included 0.8 wt.% platinum, 0.5 wt% tin, 2.7 wt% potassium and 1.3 wt% chlorine on a gamma-alumina support. The atomic ratio of potassium to platinum for this catalyst was 16.8.

The dehydrogenation catalyst was prepared by dissolving substantially pure aluminum pellets in a hydrochloric acid solution. An amount of stannic chloride was added to the resulting solution to provide a final composite containing 0.5 wt% tin and stirred to distribute the tin component evenly throughout the mixture. Hexamethylenetetraamine was added to the resulting tin mixture. The resulting tin-amine mixture was dropped into an oil bath in a manner to form spherical particles having an average particle diameter of about 1/16 inch. The spheres were aged, washed with an ammoniacal solution, dried and calcined to form a spherical gamma-alumina carrier material. The resulting spheres contained about 0.5 wt% tin in the form of tin oxide. Preparation of alumina carrier material is described in U.S. Patent No. 2,620,314 to Hoesktra, entitled "Spheroidal Alumina."

The tin-alumina composite was contacted with a deionized solution of chloroplatinic acid and hydrochloric acid (2 wt% based on alumina weight) in a rotary drier for 15 minutes at room temperature. The amount of chloroplatinic acid used was the amount necessary to incorporate 0.8 weight percent platinum into the tin-alumina composite. The solution was then heated and purged with nitrogen to remove water, resulting in a platinum-chlorine-tin-alumina composite. The incorporated chlorine was removed by heating the platinum-chlorine-tin-alumina composite to 550 °C and treating the composite with a 50/50 air/80 °C steam mixture at a gas hourly space velocity (GHSV) of 300 hr⁻¹. After treatment with the air/steam mixture, the platinum-tin-alumina composite contained less than 0.1 weight percent chlorine.

The platinum-tin-alumina composite was contacted with a solution of potassium nitrate dissolved in deionized water. The amount of potassium nitrate used was the amount necessary to incorporate 2.7 wt% of potassium in the platinum-tin-alumina composite. The water was removed from the platinum-tin-potassium-alumina composite by heating the composite to 100 °C under a purge of dry air (1000 hr⁻¹GHSV) for 0.5 hour. The temperature was raised to 525 °C and the platinum-tin-potassium alumina composite was treated with a stream of hydrochloric acid (12 cc/hr, 0.9 MHCl) and a stream of 50/50 air/80 °C steam mixture (300 hr⁻¹ GHSV) to incorporate chlorine into the platinum-tin-potassium-alumina composite. The platinum-tin-potassium-chlorine-alumina composite was dried at 525 °C under a purge of dry air (1000 hr⁻¹ GHSV). The resulting catalyst spheres had an average particle diameter of 1/16 inch and were crushed and sized into 6-20 mesh particle before testing.

Stainless steel tubing, 1 inch OD, 0.6 inch ID and 26 inches long, was used as an isomerization reactor. A thermowell extended 20 inches from the top of the stainless steel reactor tube. To load the reactor tube, the reactor tube was inverted and a piece of glass wool was transferred down the wall of the reactor tube, over the thermowell and positioned at the bottom of the reactor tube to serve as a plug for the reactor tube. Silicon carbide (20 mesh) was added to a depth of about 6 inches to the reactor tube. A second piece of glass wool was placed over the silicon carbide. A mixture of 6.0 grams of platinum-tin on alumina catalyst particles (6-20 mesh) and 45 grams of fresh silicon carbide (60-80 mesh) was added to the reactor tube in two parts. The two-part addition distributed the catalyst evenly in the reactor tube and resulted in a catalyst bed of about 10 inches in length. A third piece of glass wool was added to the top of the catalyst in the reactor tube. Silicon carbide (20 mesh) was layered onto the third piece of glass wool. A fourth piece of glass wool was positioned over the silicon carbide to serve as a plug for the bottom of the reactor tube. To monitor the temperature of the reaction at various points in the reactor tube, a multipoint thermocouple was inserted into the thermowell of the reactor tube. The temperature above, below and at three different places in the catalyst bed was monitored. The reactor tube was inverted and installed in the furnace. The reactor tube was purged with nitrogen. The reactor tube was heated to an operating temperature of 425°C over a four-hour period under flowing nitrogen (250 standard liters per hour). Once the temperature of 425°C was obtained, the reactor tube was held at the operating temperature for an additional two hours. The catalyst was presulfided by flowing a 1% mixture of hydrogen sulfide gas in hydrogen gas at 425 °C for 5 minutes through the reactor tube. After 5 minutes, the hydrogen sulfide in hydrogen gas flow was switched to a hydrogen gas flow through the reactor tube.

After presulfiding the catalyst, the reactor tube was maintained at 425 °C for eight hours. After eight hours, the reactor tube pressure was increase to 25 psig with hydrogen gas. Dodecane was pumped through the reactor tube at a flow rate of 40 g/hr at a hydrogen flow rate of 125 standard liters per hour. After four hours, flow of the dodecane stream was increased to 80 g/hr. After obtaining a flow rate of 80 g/hr, the reactor tube temperature was raised to 460 °C. The reactor tube was sampled every eight hours after obtaining the operating temperature of 460°C.

After twenty-four hours the weight percent of dodecane was 11.4 weight percent as depicted in Table 6. At a temperature of 479 °C, the conversion of dodecane to olefins was 16 weight percent after 24 hours. Of the olefins, formed 84 weight percent were mono olefins, 4.1 weight percent were aromatic compounds and 7.5 weight percent were di-olefins. Of the total amount of olefins formed, 6 percent were branched, as determined by the ¹H NMR technique previously described.

**Table 6**

| **Test Results.** | **Example 3** |
|---|---|
| Conversion (wt.%) after 24 hours on-stream at 460 °C. | 11.4 |
| Temperature required for 16 wt% conversion | 479 °C |
| Selectivity to mono olefins at 16 wt% conversion. | 84 wt% |
| Selectivity to aromatics at 16 wt% conversion. | 4.1 wt% |
| Selectivity to di-olefins at 16 wt% conversion. | 7.5 wt% |
| % Branched C₁₂ olefins in total C₁₂ olefins, (wt%) | 6 |

**EXAMPLE 4. DEHYDROGENATION-ISOMERIZATION OF DODECANE:** Dodecane was obtained from Aldrich Chemical Company and stored under nitrogen before being processed. The composition of dodecane, as assayed by gas chromatography, is tabulated in Table 5.

A dehydrogenation-isomerization catalyst was prepared in the following manner. Ammonium-ferrierite (645 grams) exhibiting a 5.4% loss on ignition and exhibiting the following properties: molar silica to alumina ratio of 62:1, surface area of 369 square meters per gram (P/Po=0.03), soda content of 480 ppm and n-hexane sorption capacity of 7.3 g per 100 g of ammonium-ferrierite was loaded into a Lancaster mix muller. CATAPAL® D alumina (91 grams) exhibiting a loss on ignition of 25.7% was added to the muller. During a five-minute mulling period, 152 milliliters of deionized water was added to the alumina/ammonium-ferrierite mixture. Next, a mixture of 6.8 grams glacial acetic acid, 7.0 grams of citric acid and 152 milliliters of deionized water was slowly added to the alumina/ammonium-ferrierite mixture in the muller to peptize the alumina. The resulting alumina/ammonium-ferrierite/acid mixture was mulled for 10 minutes. Over a period of 15 minutes, a mixture of 0.20 grams of tetraamine palladium nitrate in 153 grams of deionized water was slowly added to mulled alumina/ammonium-ferrierite/acid mixture. The resulting mixture exhibited a 90:10 ratio of zeolite to alumina and a loss on ignition of 43.5%. The zeolite/alumina mixture was shaped by extruding the mixture through a stainless steel die plate (1/16" holes) of a 2.25 inch Bonnot extruder.

Six grams of the resulting zeolite/alumina mixture was impregnated with an aqueous solution of chloroplatinic acid to incorporate 0.8 wt% platinum into the 1/16 inch extrudate, The moist zeolite/alumina platinum impregnated extrudate was dried at 125 °C for 2 hours in flowing air. The temperature was raised to a maximum temperature of 500 °C and the zeolite/alumina platinum impregnated extrudate was calcined to yield a dehydrogenation-isomerization catalyst. The calcined catalyst was crushed and sized into 6-20 mesh particles before testing.

Dodecane was dehydrogenated and isomerized using the same reactor tube design as described in Example 3. A 16.1 weight percent conversion of dodecane to olefins was observed after twenty-fours hours at 459 °C. As tabulated in Table 7, of the olefins formed 86 weight percent were mono olefins, 1.2 weight percent were aromatic compounds and 6.8 weight percent were di-olefins. Of the total amount of olefins formed, 86 percent were branched, as determined by the ¹H NMR.

**Table 7**

| **Test Results.** | **Example 4** |
|---|---|
| Conversion (wt.%) after 24 hours on-stream at 460 °C. | 16.1 |
| Temperature required for 16 wt. % conversion | 459 °C |
| Selectivity to mono olefins at 16 wt. % conversion. | 86 wt.% |
| Selectivity to aromatics at 16 wt. % conversion. | 1.2 wt.% |
| Selectivity to di-olefins at 16 wt. % conversion. | 6.8 wt. % |
| % Branched C₁₂ olefins in total C₁₂ olefins, (wt.%) | 86 |

**EXAMPLE 5: DEHYDROGENATION-ISOMERIZATION CATALYST.** A zeolite portion of a dehydrogenation-isomerization catalyst was prepared as described in Example 4. Six grams of the resulting zeolite/alumina mixture was impregnated with an aqueous solution of tetraamine palladium nitrate to incorporate 0.8 wt.% palladium into the 1/16 inch extrudates.

The moist zeolite/alumina palladium impregnated extrudate was dried at 125°C for 2 hours in flowing air. The temperature was raised to a maximum temperature of 500°C and the zeolite/alumina platinum impregnated extrudate was calcined to yield a dehydrogenation-isomerization catalyst. The calcined catalyst was carefully crushed and sized into 6-20 mesh particles before testing.

**EXAMPLE 6: DEHYDROGENATION-ISOMERIZATION CATALYST:** A dehydrogenation-isomerization catalyst was prepared by the methods described for catalyst D of U. S. Patent No. 5,648,585 to Murray et al., entitled "Process For Isomerizing Linear Olefins To Isoolefins."

Ammonium-ferrierite having a molar silica to alumina ratio of 62:1, a surface area of 369 m2/g (P/Po=0.03), a soda content of 480 ppm wt and a n-hexane sorption capacity of 7.3 grams per 100 grams of zeolite was used. The catalyst components were mulled using a Lancaster mix muller. The mulled catalyst material was extruded using a Bonnot pin barrel extruder. The binder utilized was CATAPAL® D alumina from Sasol. METHOCEL ® F4M, hydroxypropyl methylcellulose, from The Dow Chemical Company was used as an extrusion aid.

The Lancaster mix muller was loaded with 632 grams of ammonium ferrierite (LOI of 3.4%) and 92 grams of CATAPAL®D alumina (LOI of 26.2%). The alumina was blended with the ferrierite for five minutes during which time 156 mL of de-ionized water was added. A mixture of 6.8 grams of glacial acetic acid and 156 mL of de-ionized water were added slowly to the muller in order to peptize the alumina. The mixture was mix-mulled for 10 minutes. Tetraamine platinum nitrate and tetraamine palladium nitrate were added to the mix-muller in order to produce a catalyst that contained 0.25 wt% palladium and 0.55 wt% platinum. Ten grams of METHOCEL® F4M hydroxypropyl methylcellulose was added and the zeolite/alumina was mulled for 15 additional minutes. The extrudate was transferred to a Bonnot pin barrel extruder and extruded using a stainless steel die plate with 1/16 inch holes. The extrudate was dried at 120°C for 16 hours and then calcined in air at 500 °C for 2 hours. The calcined catalyst was carefully crushed and sized into 6-20 mesh particles before testing.

## Claims

1. A method for the production of alkyl aromatic hydrocarbons, comprising:
introducing a first hydrocarbon stream comprising olefins and paraffins into a dehydrogenation-isomerization unit, wherein the dehydrogenation-isomerization unit is configured to dehydrogenate at least a portion of the paraffins in the first hydrocarbon stream to olefins, and wherein the dehydrogenation-isomerization unit is further configured to isomerize at least a portion of linear olefins to branched olefins, and wherein at least a portion of the unreacted components of the first hydrocarbon stream and at least a portion of the products of the dehydrogenation and isomerization reactions form a second hydrocarbon stream, the second hydrocarbon stream comprising olefms and paraffins, and wherein at least a portion of the olefins in the second hydrocarbon stream are branched olefins; and
introducing at least a portion of the second hydrocarbon stream and aromatic hydrocarbons into an alkylation unit, wherein the alkylation unit is configured to alkylate at least a portion of the aromatic hydrocarbons with at least a portion of the olefins in the second hydrocarbon stream to produce alkyl aromatic hydrocarbons, wherein at least a portion of the produced alkyl aromatic hydrocarbons comprise a branched alkyl group.

2. The method of claim 1, wherein the first hydrocarbon stream is produced from an olefin oligomerization process or from a Fischer-Tropsch process.

3. The method of any one of claims 1 to 2, wherein the first hydrocarbon stream comprises olefins and paraffins having a carbon number from 10 to 16 or having a carbon number from 10 to 13.

4. The method of any one of claims 1 to 3, wherein the first hydrocarbon stream comprises about 80 percent paraffins or an olefin content between about 1 percent and about 50 percent of the total amount of hydrocarbons in the first hydrocarbon stream.

5. The method of any one of claims 1 to 4, wherein the dehydrogenation-isomerization unit is operated at a temperature of between about 300°C and about 500 °C.

6. The method of any one of claims 1 to 5, wherein the dehydrogenation-isomerization unit is configured to operate at a pressure of between about 0.10 atmosphere and about 15 atmospheres.

7. The method of any one of claims 1 to 6, wherein a residence time at least a portion of the unreacted hydrocarbons stream in the dehydrogenation-isomerization unit is such that the conversion level of the paraffins in the unreacted hydrocarbons stream to olefins is less than about 50 mole percent.

8. The method of any one of claims 1 to 7, wherein a portion of the branched olefins comprises an average number of branches per total olefin molecules of at least 0.7, or between about 0.7 and about 1.5, or between about 1.0 and about 1.5, or between about 0.7 and about 2.0 or less than about 2.5.

9. The method of any one of claims 1 to 8, wherein a portion of the branched olefins comprises methyl and ethyl branches.

10. The method of any one of claims 1 to 9, wherein the branched groups on the branched olefins are greater than about 50 percent methyl groups, or less than about 10 percent ethyl groups, or less than about 5 percent groups other than methyl or ethyl groups.

11. The method of any one of claims 1 to 10, wherein the branched olefins have less than about 0.5 percent or less than about 0.3 percent aliphatic quaternary carbon atoms.

12. The method of any one of claims 1 to 11, wherein the alkylation unit is configured to produce greater than about 50 percent or greater than about 85 percent of monoalkylated aromatic hydrocarbons.

13. The method of any one of claims 1 to 12, wherein a molar ratio of the aromatic hydrocarbons to the branched olefins is between about 0.1 and about 2.0 in the alkylation unit.

14. The method of any one of claims 1 to 13, wherein the alkylation unit is operated at a reaction temperature between about 30 °C and about 300 °C.

15. The method of any one of claims 1 to 14, further comprising adjusting a ratio of olefins to paraffins introduced into the alkylation unit by adding at least a portion of a third hydrocarbon stream into the alkylation unit or adding at least a portion of a third hydrocarbon stream into the alkylation unit, wherein the third hydrocarbon stream comprises greater than about 90 percent paraffins by weight

16. The method of any one of claims 1 to 14, further comprising:
adjusting a ratio of olefins to paraffins introduced into the alkylation unit by combining at least a portion of a third hydrocarbon stream with at least a portion of the second hydrocarbon stream upstream of the alkylation unit to form a combined stream; combining at least a portion of a third hydrocarbon stream with at least a portion of the second hydrocarbon stream upstream of the alkylation unit to form a combined stream, wherein the third hydrocarbon stream comprises greater than about 90 percent paraffins by weight; and
introducing the mixed stream into the alkylation unit.

17. The method of any one of claims 1 to 14, further comprising:
adjusting a ratio of olefins to paraffins introduced into the alkylation unit by combining at least a portion of a third hydrocarbon stream with at least a portion of the second hydrocarbon stream upstream of the alkylation unit to form a combined stream; by combining at least a portion of a third hydrocarbon stream with at least a portion of the second hydrocarbon stream upstream of the alkylation unit to form a combined stream, wherein the third hydrocarbon stream comprises greater than about 80, or 90, or between 85 and 95 percent paraffins by weight; by combining at least a portion of a third hydrocarbon stream with at least a portion of the second hydrocarbon stream upstream of the alkylation unit to form a combined stream, wherein the third hydrocarbon stream comprises paraffins and olefins having a carbon number from 10 to 16; by combining at least a portion of a third hydrocarbon stream with at least a portion of the second hydrocarbon stream upstream of the alkylation unit to form a combined stream, wherein the third hydrocarbon stream comprises linear olefins; by combining at least a portion of a third hydrocarbon stream with at least a portion of the second hydrocarbon stream upstream of the alkylation unit to form a combined stream, wherein at least a portion of the second hydrocarbon stream comprises branched olefins; or by combining at least a portion of a third hydrocarbon stream with at least a portion of the second hydrocarbon stream upstream of the alkylation unit to form a combined stream, wherein at least a portion of the third hydrocarbon stream comprises linear olefins and at least a portion of the second hydrocarbon stream comprises branched olefins; and
introducing the combined stream into the alkylation unit.

18. The method of any one of claims 1 to 17, wherein the branched alkyl groups of the alkyl aromatic hydrocarbons comprise 0.5 percent or less aliphatic quaternary carbon atoms, and an average number of branches per alkyl group of at least 0.7, the branches comprising methyl and ethyl branches.

19. The method of any one of claims 1 to 18, further comprising:
forming an alkylation reaction stream wherein the alkylation reaction stream comprises at least a portion of the unreacted components of the second hydrocarbon stream, at least a portion of the aromatic hydrocarbons and at least a portion of the produced alkyl aromatic hydrocarbons;
separating alkyl aromatic hydrocarbons from the alkylation reaction stream to produce an unreacted hydrocarbons stream and an alkyl aromatic hydrocarbon stream; the unreacted hydrocarbons stream comprising at least a portion of the unreacted components of the second hydrocarbon stream and aromatic hydrocarbons;
separating at least a portion of the paraffins and at least a portion of the olefms from the unreacted hydrocarbons stream to produce an aromatic hydrocarbons stream and a paraffins and unreacted olefins stream; and
introducing at least a portion of the paraffins and unreacted olefins stream into the dehydrogenation-isomerization unit.

20. The method of claim 19, wherein introducing at least a portion of the paraffins and unreacted olefins stream into the dehydrogenation-isomerization unit comprises combining at least a portion of the paraffins and unreacted olefins stream with at least a portion of the first hydrocarbon stream to produce a combined stream upstream of the dehydrogenation-isomerization unit and introducing at least a portion of the combined stream into the dehydrogenation-isomerization unit.

21. The method of any one of claims 1 to 20, wherein the dehydrogenation-isomerization unit comprises a dehydrogenation-isomerization catalyst configured to catalyze both dehydrogenation reactions and isomerization reactions in the dehydrogenation-isomerization unit, the catalyst comprising a hydrogen form of a zeolite have a ferrierite isotypic framework structure, a binder, a coke-oxidizing compound, and a paraffin dehydrogenation promoting compound.

22. The method of claim 21, wherein the coke-oxidizing compound comprises chrome oxide, iron oxide or noble metals or mixtures thereof, a noble metal; or platinum, palladium, iridium, ruthenium, osmium or rhodium or mixtures thereof.

23. The method of any one of claims 21 to 22, wherein the paraffin dehydrogenation promoting compound is platinum or a noble metal.

24. The method of any one of claims 21 to 23, wherein the binder is selected from natural clays, alumina and silica-alumina or is alumina.

25. The method of any one of claims 1 to 24, wherein the dehydrogenation-isomerization unit comprises a plurality of zones, wherein the plurality of zones comprises a first reaction zone and a second reaction zone, wherein the first reaction zone is configured to dehydrogenate at least a portion of paraffins to olefins, and wherein the second reaction zone is configured to isomerize at least a portion of linear olefins to branched olefins, and wherein at least a portion of the unreacted components of the first hydrocarbon stream and at least a portion of the products of the dehydrogenation and isomerization reactions form a second hydrocarbon stream.

26. The method of any one of claims 1 to 24, wherein the dehydrogenation-isomerization unit comprises a plurality of zones, wherein the plurality of zones comprises a first reaction zone and a second reaction zone, wherein the first reaction zone is configured to dehydrogenate at least a portion of paraffins to olefins, wherein the second reaction zone is configured to isomerize at least a portion of linear olefins to branched olefins; and wherein the first reaction zone is operated at a temperature of between about 300 °C and about 600 °C.

27. The method of any one of claims 1 to 24, wherein the dehydrogenation-isomerization unit comprises a plurality of zones, wherein the plurality of zones comprises a first reaction zone and a second reaction zone, wherein the first reaction zone is configured to dehydrogenate at least a portion of paraffins to olefins, wherein the second reaction zone is configured to isomerize at least a portion of linear olefins to branched olefins; and wherein the first reaction zone is operated at a temperature of between about 350 °C and about 550 °C.

28. The method of any one of claims 1 to 24, wherein the dehydrogenation-isomerization unit comprises a plurality of zones, wherein the plurality of zones comprises a first reaction zone and a second reaction zone, wherein the first reaction zone is configured to dehydrogenate at least a portion of paraffins to olefins, wherein the second reaction zone is configured to isomerize at least a portion of linear olefins to branched olefins; and wherein the first reaction zone is operated at a total reaction pressure between about 1.0 atmosphere and about 5.0 atmospheres.

29. The method of any one of claims 1 to 24, wherein the dehydrogenation-isomerization unit comprises a plurality of zones, wherein the plurality of zones comprises a first reaction zone and a second reaction zone, wherein the first reaction zone is configured to dehydrogenate at least a portion of paraffins to olefins, wherein the second reaction zone is configured to isomerize at least a portion of linear olefins to branched olefins; and wherein a residence time of at least a portion of the first hydrocarbon stream in the first reaction zone is such that the conversion level of the paraffins to olefins is less than about 50 mole percent.

30. The method of any one of claims 1 to 24, wherein the dehydrogenation-isomerization unit comprises a plurality of zones, wherein the plurality of zones comprises a first reaction zone and a second reaction zone, wherein the first reaction zone is configured to dehydrogenate at least a portion of paraffins to olefins, wherein the second reaction zone is configured to isomerize at least a portion of linear olefins to branched olefins; and wherein the second reaction zone is operated at a temperature range of about 200 °C to about 500 °C.

31. The method of any one of claims 1 to 24, wherein the dehydrogenation-isomerization unit comprises a plurality of zones, wherein the plurality of zones comprises a first reaction zone and a second reaction zone, wherein the first reaction zone is configured to dehydrogenate at least a portion of paraffins to olefins, wherein the second reaction zone is configured to isomerize at least a portion of linear olefins to branched olefins; and wherein the second reaction zone is operated at a hydrocarbon partial pressure between about 0.1 atmosphere and about 10 atmospheres.

32. The method of any one of claims 1 to 24, wherein the dehydrogenation-isomerization unit comprises a plurality of zones, wherein the plurality of zones comprises a first reaction zone and a second reaction zone, wherein the first reaction zone is configured to dehydrogenate at least a portion of paraffins to olefins, wherein the second reaction zone is configured to isomerize at least a portion of linear olefins to branched olefins and wherein the second reaction zone is operated at a hydrocarbon partial pressure between about 0.5 atmosphere and about 2 atmospheres.

33. The method of any one of claims 1 to 32, further comprising introducing at least a portion of the first hydrocarbon stream exiting the first reaction zone into a heat exchanger, wherein the heat exchanger is configured to remove heat from a portion of the first hydrocarbon stream before it enters the second reaction zone.

34. The method of any one of claims 1 to 33, wherein the dehydrogenation-isomerization unit comprises a stacked bed catalyst configuration, wherein the stacked bed catalyst comprises a dehydrogenation catalyst and an isomerization catalyst.

35. The method of any one of claims I to 28, further comprising introducing hydrogen into the first hydrocarbon stream.

36. The method of any one of claims 1 to 35, further comprising introducing at least a portion of the alkyl aromatic hydrocarbons stream into a sulfonation unit, wherein the sulfonation unit is configured to sulfonate at least a portion of the alkyl aromatic hydrocarbons in the alkyl aromatic hydrocarbons stream to produce alkyl aromatic sulfonates and wherein at least a portion of the alkyl aromatic sulfonates produced comprise branched alkyl aromatic sulfonates.

## Patentansprüche

1. Eine Methode zur Erzeugung von alkylaromatischen Kohlenwasserstoffen, beinhaltend:
das Einführen eines ersten Stromes von Kohlenwasserstoffen zusammengesetzt aus Olefinen und Paraffinen in eine Dehydrierungs-Isomerisierungseinheit, wobei die Dehydrierungs-Isomerisierungseinheit so gestaltet ist, dass wenigstens ein Teil der Paraffine im ersten Kohlenwasserstrom zu Olefinen dehydrogeniert werden, und wobei die Dehydrierungs-Isomerisierungseinheit so gestaltet ist, dass außerdem wenigstens ein Teil der unverzweigten Olefine des ersten Kohlenwasserstroms in verzweigte Olefine isomerisiert werden, und wobei wenigstens ein Teil der nicht umgesetzten Komponenten des ersten Kohlenwasserstoffstroms und wenigstens ein Teil der Produkte der Dehydrogenierungs- und Isomerisierungsreaktionen einen zweiten Kohlenwasserstoffstrom bilden;
das Einführen von wenigstens einem Teil des zweiten Kohlenwasserstoffstroms und aromatischen Kohlenwasserstoffen in eine Alkylierungseinheit, wobei die Alkylierungseinheit zur Alkylierung von wenigstens einem Teil der aromatischen Kohlenwasserstoffen mit wenigstens einem Teil der Olefine des zweiten Kohlenwasserstroms zu alkylaromatischen Kohlenwasserstoffe gestaltet ist, und wobei wenigstens ein Teil der erzeugten alkylaromatischen Kohlenwasserstoffe eine verzweigte Alkylgruppe umfasst.

2. Die Methode von Anspruch 1, wobei der erste Strom von Kohlenwasserstoffen von einem Olefin-Oligomerisationsverfahren oder einem Fischer-Tropschverfahren ausgeht.

3. Die Methode einer der Ansprüche von 1 oder 2, wobei der erste Kohlenwasserstoffstrom Olefine und Paraffine umfasst mit einer Kohlenstoffzahl zwischen 10 und 13 oder zwischen 10 und 16.

4. Die Methode einer der Ansprüche von 1 bis 3, wobei der erste Kohlenwasserstoffstrom 80% Paraffine enthält, oder einen Olefingehalt zwischen ungefähr 1 und 50 Prozent der Gesamtmenge an Kohlenwasserstoffen im ersten Kohlenwasserstoffstrom enthält.

5. Die Methode einer der Ansprüche von 1 bis 4, bei der die Dehydrogenierungs-Isomerisierungseinheit bei einer Reaktionstemperatur zwischen ungefähr 300°C und ungefähr 500°C betrieben wird.

6. Die Methode einer der Ansprüche von 1 bis 5, bei der die Dehydrogenierungs-Isomerisierungseinheit bei einem Reaktionsdruck zwischen ungefähr 0,1 Atmosphären und ungefähr 15 Atmosphären betrieben wird.

7. Die Methode einer der Ansprüche von 1 bis 6, wobei die Verbleibzeit wenigstens eines Teils des nicht umgesetzten Kohlenwasserstoffsstroms in der Dehydrogenierungs-Isomerisierungseinheit so ist, dass der Umsatz der Paraffine in Zusammensetzung des nicht umgesetzten Kohlenwasserstoffsstroms in Olefine unter ungefähr 50 Mol % liegt.

8. Die Methode einer der Ansprüche von 1 bis 7, wobei ein Teil der verzweigten Olefine eine Durchschnittszahl von Verzweigungen pro Gesamtolefinmolekül von mindestens 0.7, zwischen ungefähr 0.7 und ungefähr 1.5, zwischen ungefähr 0.7 und ungefähr 2.0, zwischen ungefähr 1.0 und ungefähr 1.5, oder weniger als ungefähr 2.5 enthält.

9. Die Methode einer der Ansprüche von 1 bis 8, wobei bei der wenigstens ein Teil der verzweigten Olefine Methyl- und Ethylverzweigungen enthält.

10. Die Methode einer der Ansprüche von 1 bis 9, wobei die verzweigten Gruppen der verzweigten Olefine zu mehr als ungefähr 50% aus Methylgruppen, zu weniger als ungefähr 10% aus Ethylgruppen, oder zu weniger als ungefähr 5% aus Gruppen die weder Ethyl noch Methylgruppen sind, bestehen.

11. Die Methode einer der Ansprüche von 1 bis 10, wobei die verzweigten Olefine weniger als ungefähr 0,5% aliphatischer quaternärer Kohlenstoffatome oder weniger als ungefähr 3% aliphatischer quaternärer Kohlenstoffatome beinhalten.

12. Die Methode einer der Ansprüche von 1 bis 11, wobei die Alkylierungseinheit gestaltet ist, um mehr als ungefähr 50% oder mehr als ungefähr 85% aromatischer Monoalkylkohlenwasserstoffe zu erzeugen.

13. Die Methode einer der Ansprüche von 1 bis 12, wobei in der Alkylierungseinheit das Molverhältnis zwischen den aromatischen Kohlenwasserstoffen und den verzweigten Olefinen zwischen ungefähr 0.1 und ungefähr 2.0 liegt.

14. Die Methode einer de Ansprüche von 1 bis 11, wobei die Alkylierungseinheit bei einer Reaktionstemperatur betreiben wird, die zwischen ungefähr 30 °C und ungefähr 300 °C liegt.

15. Die Methode einer der Ansprüche von 1 bis 14, ausserdem umfassend das Angleichen des Verhältnisses zwischen Olefinen und Paraffinen die in die Alkylierungseinheit eingeführt werden, durch Zufügen wenigstens eines Teiles eines dritten Kohlenwasserstoffstroms in die Alkylierungseinheit, oder durch Zufügen wenigstens eines Teiles eines dritten Kohlenwasserstoffstroms in die Alkylierungseinheit, wobei der dritte Kohlenwasserstoffstrom mehr als 90 Gewichtprozent an Paraffinen befasst.

16. Die Methode einer der Ansprüche von 1 bis 15, die ausserdem das Verhältnis von Olefinen zu Paraffinen, welche in die Alkylierungseinheit eingeführt werden, angleicht, durch Zusammenfügen wenigstens eines Teils eines dritten Kohlenwasserstroms mit wenigstens eines Teils eines zweiten Kohlenwasserstroms vor dem Einbringen in die Alkylierungseinheit zu einen zusammengefügten Storm; durch Zusammenfügen wenigstens eines Teils eines dritten Kohlenwasserstroms mit wenigstens eines Teils eines zweiten Kohlenwasserstroms vor dem Einbringen in die Alkylierungseinheit zu einen zusammengefügten Strom, wobei der dritte Kohlenwasserstoffstrom mehr als 90 Gewichtprozent an Paraffinen befasst; und
Einbringen in die Alkyliertungseinheit.

17. Die Methode einer der Ansprüche von 1 bis 16, die ausserdem das Angleichen des Verhältnisses von Olefinen zu Paraffinen, welche in die Alkylierungseinheit eingeführt werden, umfasst, durch Zusammenfügen wenigstens eines Teils eines dritten Stroms von Kohlenwasserstoffen mit wenigstens einem Teil eines zweiten Stroms von Kohlenwasserstoffen vor dem Einbringen in die Alkylierungseinheit, wobei der dritte Kohlenwasserstoffstrom mehr als ungefähr 80, mehr als ungefähr 90 oder zwischen 85 und 95% Gewicht an Paraffinen umfasst;
durch Zusammenfügen von wenigstens einem Teil eines dritten Kohlenwasserstoffstroms mit wenigstens einem Teil eines zweiten Kohlenwasserstoffstroms vor Eintritt in die Alkylierungseinheit unter Bildung eines zusammengeführten Stroms, wobei von wenigstens einem Teil des dritten Kohlenwasserstoffstroms Paraffine und Olefine umfasst die 10 bis 16 Kohlenstoffe enthalten;
durch Zusammenfügen von wenigstens einem Teil eines dritten Kohlenwasserstoffstroms mit wenigstens einem Teil eines zweiten Kohlenwasserstoffstroms vor Eintritt in die Alkylierungseinheit unter Bildung eines zusammengeführten Stroms, wobei wenigstens ein Teil des dritten Kohlenwasserstoffstrom unverzweigte Olefine enthält;
durch Zusammenfügen von wenigstens einem Teil eines dritten Kohlenwasserstoffstroms mit wenigstens einem Teil eines zweiten Kohlenwasserstoffstroms vor Eintritt in die Alkylierungseinheit unter Bildung eines zusammengeführten Stroms, wobei wenigstens ein Teil des zweiten Kohlenwasserstoffstroms verzweigte Olefine enthält;
durch Zusammenfügen von wenigstens einem Teil eines dritten Kohlenwasserstoffstroms mit wenigstens einem Teil eines zweiten Kohlenwasserstoffstroms vor Eintritt in die Alkylierungseinheit unter Bildung eines zusammengeführten Stroms, wobei wenigstens ein Teil des dritten Kohlenwasserstoffstrom unverzweigte Olefine enthält und wobei wenigstens ein Teil des zweiten Kohlenwasserstoffstroms verzweigte Olefine enthält;
und das Eingbringen der zusammengefügten Stroms in die Alkylierungseinheit.

18. Die Methode einer der Ansprüche von 1 bis 17, wobei die verzweigten Alkylgruppen der alkylaromatischen Kohlenwasserstoffe 0,5% oder weniger aliphatischer quaternärer Kohlenstoffatome enthalten, und eine gemittelte Anzahl an Verzweigungen pro Alkylgruppe von mindestens 0.7 beträgt, und die Verzweigungen Methyl- und Ethylverzweigungen enthalten.

19. Die Methode einer der Ansprüche von 1 bis 18, welche ausserdem die Bildung eines Stroms zur Alkylierungsreaktion vorsieht und der Strom für die Alkylierungsreaktion mindestens einen Teil der Komponente ohne Reaktion des zweiten Stroms von Kohlenwasserstoffen, mindestens einen Teil der aromatischen Kohlenwasserstoffe und mindestens einen Teil der erzeugten alkylaromatischen Kohlenwasserstoffe, umfasst;
Abtrennung der alkylaromatischen Kohlenwasserstoffe vom Strom für die Alkylierungsreaktion, um einen Strom von nicht umgesetzten Kohlenwasserstoffen und einen Strom von alkylaromatischen Kolenwasserstoffen zu erzeugen, wobei Strom von nicht umgesetzten Kohlenwasserstoffen mindestens einen Teil der nicht umgesetzten Komponente des zweiten Stroms von Kohlenwasserstoffen und aromatischen Kohlenwasserstoffen umfasst;
Abtrennung von mindestens einem Teil der Paraffine und mindestens einem Teil der Olefine des Stroms nicht umgesetzter Kohlenwasserstoffe, um einen Strom von aromatischen Kohlenwasserstoffen und einen Strom von Praffinen und Olefinen ohne Reaktion zu erzeugen;
Einführung von mindestens einem Teil des Stroms von nicht umgesetzten Paraffinen und Olefinen in die Isomerisierungs-Dehydrierungseinheit.

20. Die Methode des Anspruchs 19, wobei die Einführung von mindestens einem Teil des Stroms von nicht umgesetzten Paraffinen und Olefinen in die Isomerisierungs-Dehydrierungseinheit durch die Zusammenfügung von mindestens einem Teil des Stroms von nicht umgesetzten Paraffinen und Olefinen mit mindestens einem Teil des ersten Stroms von Kohlenwasserstoffen, um einen zusammengefügten Strom vor der Isomerisierungs-Dehydrierungseinheit zu erzeugen, und das Einführen mindestens einen Teil des zusammengefügten Stroms in die Isomerisierungs-Dehydrierungseinheit, geschieht.

21. Die Methode einer der Ansprüche von 1 bis 20, wobei die Isomerisierungs-Dehydrierungseinheit einen Isomerisierung-Dehydrierung Katalysator enthält, um sowohl die Dehydrierungsreaktion, als auch die Isomerisierungsreaktion in der Isomerisierungs-Dehydrierungseinheit zu katalysieren, wobei der Katalysator eine Form von wasserstoffhaltigem Zeolith mit der isotypischen Rahmenstruktur der Ferrierite, ein Bindemittel, eine Verbindung zum Oxidieren von Koks, und eine Paraffin-Dehydrierungskomponente beinhaltet.

22. Die Methode des Anspruchs 21 , wobei die Verbindung zum Oxidieren von Koks Chromoxyd, Eisenoxyd oder Edelmetalle oder Mischungen derselben, ein Edelmetal; oder Platin, Palladium, Iridium, Ruthenium, Osmium oder Rhodium, oder Mischungen derselben beinhaltet.

23. Die Methode einer der Ansprüche von 21 bis 22, wobei die Paraffin-Dehydrierungskomponente platin oder ein Edelmetall ist. das Bindemittel aus natürlicher Tonerde, Tonerde, Kieselerde oder Alaunerde gewählt wird.

24. Die Methode einer der Ansprüche von 21 bis 23, bei welcher das Bindematerial aus natürlicher Tonerde, Alumina, Silica-Alumina oder Silica gewählt wird.

25. Die Methode von einer der Ansprüche von 1 bis 24, bei der die Isomerisierungs-Dehydrogenierungseinheit eine Pluralität von Zonen umfasst, und wobei diese Pluralität von Zonen eine erste Reaktionszone und eine zweite Reaktionszone umfasst, wobei die erste Reaktionszone zur Dehydrierung von wenigstens einem Teil der Paraffine in Olefine gestaltet ist, wobei die zweite Reaktionszone zur Isomerisierung von mindestens einem Teil der unverzweigten Olefine in verzweigte Olefine gestaltet ist und wobei wenigstens ein Teil der nichtumgesetzten Komponente des ersten Stroms von Kohlenwasserstoffen und wenigstens ein Teil der Produkte der Dehydrogenierungsreaktion und Isomerisierungsreaktion einen zweiten Strom von Kohlenwasserstoffen bildet.

26. Die Methode von einer der Ansprüche von 1 bis 24, bei der die Isomerisierungs-Dehydrogenierungseinheit eine Pluralität von Zonen umfasst, und wobei diese Pluralität von Zonen eine erste Reaktionszone und eine zweite Reaktionszone umfasst, wobei die erste Reaktionszone zur Dehydrierung von wenigstens einem Teil der Paraffine in Olefine gestaltet ist, wobei die zweite Reaktionszone zur Isomerisierung von mindestens einem Teil der unverzweigten Olefine in verzweigte Olefine gestaltet ist und wobei die erste Reaktionszone bei einer Temperatur zwischen ungefähr 300 °C und ungefähr 600 °C betrieben wird.

27. Die Methode von einer der Ansprüche von 1 bis 24, bei der die Isomerisierungs-Dehydrogenierungseinheit eine Pluralität von Zonen umfasst, und wobei diese Pluralität von Zonen eine erste Reaktionszone und eine zweite Reaktionszone umfasst, wobei die erste Reaktionszone zur Dehydrierung von wenigstens einem Teil der Paraffine in Olefine gestaltet ist, wobei die zweite Reaktionszone zur Isomerisierung von mindestens einem Teil der unverzweigten Olefine in verzweigte Olefine gestaltet ist und wobeidie erste Reaktionszone bei einer Temperatur zwischen ungefähr 350 °C und ungefähr 550 °C betrieben wird.

28. Die Methode von einer der Ansprüche von 1 bis 24, bei der die Isomerisierungs-Dehydrogenierungseinheit eine Pluralität von Zonen umfasst, und wobei diese Pluralität von Zonen eine erste Reaktionszone und eine zweite Reaktionszone umfasst, wobei die erste Reaktionszone zur Dehydrierung von wenigstens einem Teil der Paraffine in Olefine gestaltet ist, wobei die zweite Reaktionszone zur Isomerisierung von mindestens einem Teil der unverzweigten Olefine in verzweigte Olefine gestaltet ist und wobei die erste Reaktionszone bei einem Gesamtreaktionsdruck zwischen ungefähr 1.0 Athmosphäre und ungefähr 5,0 Athmosphären betrieben wird.

29. Die Methode von einer der Ansprüche von 1 bis 24, bei der die Isomerisierungs-Dehydrogenierungseinheit eine Pluralität von Zonen umfasst, und wobei diese Pluralität von Zonen eine erste Reaktionszone und eine zweite Reaktionszone umfasst, wobei die erste Reaktionszone zur Dehydrierung von wenigstens einem Teil der Paraffine in Olefine gestaltet ist, wobei die zweite Reaktionszone zur Isomerisierung von mindestens einem Teil der unverzweigten Olefine in verzweigte Olefine gestaltet ist und wobei die Verbleibzeit von wenigstens einem Teil des ersten Stroms von Kohlenwasserstoffen in der ersten Reaktionszone so ist, dass der Umsatz der Paraffine in Olefine niedriger als ungefähr 50 Mol % liegt.

30. Die Methode von einer der Ansprüche von 1 bis 24, bei der die Isomerisierungs-Dehydrogenierungseinheit eine Pluralität von Zonen umfasst, und wobei diese Pluralität von Zonen eine erste Reaktionszone und eine zweite Reaktionszone umfasst, wobei die erste Reaktionszone zur Dehydrierung von wenigstens einem Teil der Paraffine in Olefine gestaltet ist, wobei die zweite Reaktionszone zur Isomerisierung von mindestens einem Teil der unverzweigten Olefine in verzweigte Olefine gestaltet ist, und wobei die zweite Reaktionszone bei einer Temperatur zwischen ungefähr 200 °C und ungefähr 500 °C funktioniert.

31. Die Methode von einer der Ansprüche von 1 bis 24, bei der die Isomerisierungs-Dehydrogenierungseinheit eine Pluralität von Zonen umfasst, und wobei diese Pluralität von Zonen eine erste Reaktionszone und eine zweite Reaktionszone umfasst, wobei die erste Reaktionszone zur Dehydrierung von wenigstens einem Teil der Paraffine in Olefine gestaltet ist, wobei die zweite Reaktionszone zur Isomerisierung von mindestens einem Teil der unverzweigten Olefine in verzweigte Olefine gestaltet ist und wobei die zweite Reaktionszone mit einem Kohlenwasserstoffpartialdruck zwischen ungefähr 0.1 Atmosphären und ungefähr 10 Atmosphären betrieben wird.

32. Die Methode einer der Ansprüche von 1 bis 24 wo die Isomerisierungs-Dehydrogenierungseinheit eine Pluralität von Zonen umfasst, und wobei diese Pluralität von Zonen eine erste Reaktionszone und eine zweite Reaktionszone umfasst, wobei die erste Reaktionszone zur Dehydierung von wenigstens einem Teil der Paraffine in Olefine gestaltet ist, und die zweite Zone zur Isomerisierungs von wenigstens einem Teil der unverzweigten Olefine in verzweigte Olefine gestaltet ist, und wobei die zweite Reaktionszone unter einem Kohlenwasserstoffpartialdruck zwischen ungefähr 0.5 Atmosphäre und ungefähr 2 Atmosphäre, betrieben wird

33. Die Methode einer der Ansprüche von 1 bis 32, worin wenigstens ein Teil des ersten Stroms von Kohlenwasserstoffen nach dem Verlassen der ersten Reaktionszone in einen Wärmeaustauscher eingeführt wird, wobei der Wärmeaustauscher zum Entfernen der Wärme eines Teils des ersten Kohlenwasserstoffstroms, bevor dieser in die zweite Reaktionszone eintritt, gestaltet ist.

34. Die Methode einer der Ansprüche von 1 bis 33, wo die Isomerisierungs-Dehydrognierungseinheit einen Festbettkatalysator enthält, wobei der Festbettkatalysator einen Dehydrierungskatalysator und einen Isomerisierungskatalysator enthält.

35. Die Methode einer der Ansprüche von 1 bis 28, die außerdem das Einführen von Wasserstoff in den ersten Strom von Kohlenwasserstoffen umfasst.

36. Die Methode einer der Ansprüche von 1 bis 35, die außerdem die Einführung von wenigstens einem Teil des alkylaromatischen Kohlenwasserstoffstroms in eine Sulfonierungsseinheit umfasst, wobei die Sulfonierungsseinheit zur Sulfonierung von wenigstens einem Teil der alkylaromatischen Kohlenwasserstoffe ausgestattet ist, um alkylaromatische Sulfonate zu erzeugen, wobei zumindest ein Teil der alkylaromatischen Sulfonate verzweigte alkylaromatischen Sulfonate umfassen.

## Revendications

1. Une méthode pour produire d'hydrocarbures alkyl aromatiques qui comprend:
introduire un premier courant d'hydrocarbures composé d'oléfines et de paraffines dans une unité de déshydrogénation-isomérisation, où l'unité de déshydrogénation-isomérisation est configurée pour déshydrogéner au moins une partie des paraffines du premier courant d'hydrocarbures en oléfines, et où en outre l'unité de déshydrogénation-isomérisation est configurée pour isomériser au moins une partie des oléfines linéaires en oléfines ramifiées, et où au moins une partie des composants non-réagi du premier courant d'hydrocarbures et au moins une partie des produits des réactions de déshydrogénation et isomérisation forment un second courant d'hydrocarbures, en comprenant le second courant d'hydrocarbures oléfines et paraffines, et où au moins une partie du second courant d'hydrocarbures est constituée par oléfines ramifiées; et
introduire au moins une partie du second courant d'hydrocarbures et hydrocarbures aromatiques dans une unité d'alkylation, où l'unité d'alkylation est configurée pour alkyler au moins une partie des hydrocarbures aromatiques avec au moins une partie des oléfines du second courant d'hydrocarbures, de manière de produire des hydrocarbures alkyl aromatiques, où au moins une partie des hydrocarbures alkyl aromatiques produits comprend un groupe alkyl ramifié.

2. La méthode de la revendication 1, où le premier courant d'hydrocarbures se produit à partir d'un processus d'oligomérisation d'oléfines ou d'un processus de Fischer-Tropsch.

3. La méthode de n'importe quelles des revendications 1 à 2, où le premier courant d'hydrocarbures comprend des oléfines et des paraffines ayant un nombre de carbones de 10 à 16 ou ayant un nombre de carbones de 10 à 13.

4. La méthode de n'importe quelles des revendications 1 à 3, où le premier courant d'hydrocarbures comprend environ 80 pour cent de paraffines, ou a un contenu d'oléfines entre environ 1 pour cent et environ 50 pour cent de la quantité totale d'hydrocarbures dans le premier courant d'hydrocarbures.

5. La méthode de n'importe quelles des revendications 1 à 4, où l'unité de déshydrogénation-isomérisation est opérée à une température comprise entre environ 300 °C et environ 500°C.

6. La méthode de n'importe quelles des revendications 1 à 5, où l'unité de déshydrogénation-isomérisation est configurée pour opérer à une pression comprise entre environ 0.10 atmosphère et environ 15 atmosphères.

7. La méthode de n'importe quelles des revendications 1 à 6, où le temps de permanence d'au moins une partie du courant d'hydrocarbures non-réagi dans l'unité de déshydrogénation-isomérisation est tel, que le niveau de conversion des paraffines du courant d'hydrocarbures non-réagi en oléfines est inférieur à environ 50 pour cent en moles.

8. La méthode de n'importe quelles des revendications 1 à 7, où une partie des oléfines ramifiées comprend un nombre moyen de branches par molécules totales d'oléfine d'au moins 0.7, ou compris entre environ 0.7 et environ 1.5, ou compris entre environ 1.0 et environ 1.5, ou compris entre environ 0.7 et environ 2.0 ou inférieur à environ 2.5.

9. La méthode de n'importe quelles des revendications 1 à 8, où une partie des oléfines ramifiées comprend des branches méthyle et éthyle.

10. La méthode de n'importe quelles des revendications 1 à 9, où les groupes ramifiés en oléfines ramifiées sont plus d'environ 50 pour cent groupes méthyle, ou moins d'environ 10 pour cent groupes éthyle, ou moins d'environ 5 pour cent groupes n'étant ni méthyle ni éthyle.

11. La méthode de n'importe quelles des revendications 1 à 10, où les oléfines ramifiées ont moins d'environ 0.5 pour cent ou moins d'environ 0.3 pour cent d'atomes de carbone aliphatiques quaternaires.

12. La méthode de n'importe quelles des revendications 1 à 11, où l'unité d'alkylation est configurée pour produire plus d'environ 50 pour cent ou plus d'environ 85 pour cent d'hydrocarbures aromatiques monoalkylés.

13. La méthode de n'importe quelles des revendications 1 à 12, où dans l'unité d'alkylation la relation molaire entre les hydrocarbures aromatiques et les oléfines ramifiées est comprise entre environ 0.1 et environ 2.0.

14. La méthode de n'importe quelles des revendications 1 à 13, où l'unité d'alkylation est opérée à une température de réaction comprise entre environ 30°C et environ 300 °C.

15. La méthode de n'importe quelles des revendications 1 à 14, qui comprend en outre ajuster la proportion d'oléfines et paraffines qu'on introduit dans l'unité d'alkylation, en ajoutant au moins une partie d'un troisième courant d'hydrocarbures dans l'unité d'alkylation ou en ajoutant au moins une partie d'un troisième courant d'hydrocarbures dans l'unité d'alkylation, où le troisième courant d'hydrocarbures comprend plus d'environ 90 pour cent en poids de paraffines.

16. La méthode de n'importe quelles des revendications 1 à 14, qui comprend en outre:
ajuster la proportion d'oléfines et paraffines qui sont introduites dans l'unité d'alkylation en combinant au moins une partie d'un troisième courant d'hydrocarbures avec au moins une partie du second courant d'hydrocarbures en amont de l'unité d'alkylation, de manière de former un courant combiné; combiner au moins une partie d'un troisième courant d'hydrocarbures avec au moins une partie du second courant d'hydrocarbures en amont de l'unité d'alkylation, de sorte de former un courant combiné, où le troisième courant d'hydrocarbures comprend plus d'environ 90 pour cent en poids de paraffines; et
introduire le courant combiné dans l'unité d'alkylation.

17. La méthode de n'importe quelles des revendications 1 à 14, qui comprend en outre:
ajuster la proportion d'oléfines et paraffines qui sont introduites dans l'unité d'alkylation, en combinant au moins une partie d'un troisième courant d'hydrocarbures avec au moins une partie du second courant d'hydrocarbures en amont de l'unité d'alkylation, de façon de former un courant combiné; en combinant au moins une partie d'un troisième courant d'hydrocarbures avec au moins une partie du second courant d'hydrocarbures en amont de l'unité d'alkylation, de manière de former un courant combiné, où le troisième courant d'hydrocarbures comprend plus d'environ 80, ou 90, ou entre 85 et 95 pour cent en poids de paraffines; en combinant au moins une partie d'un troisième courant d'hydrocarbures avec au moins une partie du second courant d'hydrocarbures en amont de l'unité d'alkylation, de manière de former un courant combiné, où le troisième courant d'hydrocarbures comprend des paraffines et des oléfines ayant un nombre de carbones de 10 à 16; en combinant au moins une partie d'un troisième courant d'hydrocarbures avec au moins une partie du second courant d'hydrocarbures en amont de l'unité d'alkylation, de manière de former un courant combiné, où le troisième courant d'hydrocarbures comprend des oléfines linéaires; en combinant au moins une partie d'un troisième courant d'hydrocarbures avec au moins une partie du second courant d'hydrocarbures en amont de l'unité d'alkylation, de manière de former un courant combiné, où au moins une partie du second courant d'hydrocarbures comprend des oléfines ramifiées; ou en combinant au moins une partie d'un troisième courant d'hydrocarbures avec au moins une partie du second courant d'hydrocarbures en amont de l'unité d'alkylation, de manière de former un courant combiné, où au moins une partie du troisième courant d'hydrocarbures comprend des oléfines linéaires et au moins une partie du second courant d'hydrocarbures comprend des oléfines ramifiées; et
introduire le courant combiné dans l'unité d'alkylation.

18. La méthode de n'importe quelles des revendications 1 à 17, où les groupes alkyl ramifiés des hydrocarbures alkyl aromatiques comprennent 0.5 pour cent ou moins d'atomes de carbone aliphatiques quaternaires, et un nombre moyen de branches par groupe alkyl d'au moins 0.7, où les branches comprennent des branches méthyle et éthyle.

19. La méthode de n'importe quelles des revendications 1 à 18, qui comprend en outre:
former un courant pour une réaction d'alkylation, où le courant pour la réaction d'alkylation comprend au moins une partie des composants non-réagi du second courant d'hydrocarbures, au moins une partie des hydrocarbures aromatiques et au moins une partie des hydrocarbures alkyl aromatiques produits;
séparer les hydrocarbures alkyl aromatiques du courant pour la réaction d'alkylation, de manière de produire un courant d'hydrocarbures non-réagi et un courant d'hydrocarbures alkyl aromatiques, comprenant le courant d'hydrocarbures non-réagi, au moins une partie des composants non-réagi du second courant hydrocarbures et hydrocarbures aromatiques;
séparer au moins une partie des paraffines et au moins une partie des oléfines du courant d'hydrocarbures non-réagi, de manière de produire un courant d'hydrocarbures aromatiques et un courant de paraffines et oléfines non-réagi; et
introduire au moins une partie du courant de paraffines et oléfines non-réagi dans l'unité de déshydrogénation-isomérisation.

20. La méthode de la revendication 19, où introduire au moins une partie du courant de paraffines et oléfines non-réagi dans l'unité de déshydrogénation-isomérisation, comprend combiner au moins une partie du courant de paraffines et oléfines non-réagi avec au moins une partie du premier courant d'hydrocarbures, de manière de produire un courant combiné en amont de l'unité de déshydrogénation-isomérisation et d'introduire au moins une partie du courant combiné dans l'unité de déshydrogénation-isomérisation.

21. La méthode de n'importe quelles des revendications 1 à 20, où l'unité de déshydrogénation-isomérisation comprend un catalyseur de la déshydrogénation-isomérisation configuré pour catalyser tant la réaction de déshydrogénation, comme la réaction d'isomérisation dans l'unité de déshydrogénation-isomérisation, où le catalyseur comprend une forme de zéolithe hydrogénée avec une structure cadre isotypique de la ferrierite, un agglutinant, un composé oxydant du coke, et un composé promoteur de la déshydrogénation de la paraffine.

22. La méthode de la revendication 21, où le composé oxydant du coke comprend de l'oxyde de chrome, oxyde de fer ou métaux nobles ou mélanges de ces derniers, un métal noble; ou platine, palladium, iridium, ruthénium, osmium ou rhodium ou mélanges de ces derniers.

23. La méthode de n'importe quelles des revendications 21 à 22, où le composé promoteur de la déshydrogénation de la paraffine est le platine ou un métal noble.

24. La méthode de n'importe quelles des revendications 21 à 23, où l'agglutinant est choisi entre les argiles naturelles, l'alumine et le silice-alumine ou bien c'est l'alumine.

25. La méthode de n'importe quelles des revendications 1 à 24, où l'unité de déshydrogénation-isomérisation comprend une pluralité de zones, et cette pluralité de zones comprend une première zone de réaction et une seconde zone de réaction, où la première zone de réaction est configurée pour déshydrogéner au moins une partie des paraffines en oléfines, et où la seconde zone de réaction est configurée pour isomériser au moins une partie des oléfines linéaires en oléfines ramifiées, et où au moins une partie des composants non-réagi du premier courant d'hydrocarbures et au moins une partie des produits des réactions de déshydrogénation et isomérisation forment un second courant d'hydrocarbures.

26. La méthode de n'importe quelles des revendications 1 à 24, où l'unité de déshydrogénation-isomérisation comprend une pluralité de zones, et cette pluralité de zones comprend une première zone de réaction et une seconde zone de réaction, où la première zone de réaction est configurée pour déshydrogéner au moins une partie des paraffines en oléfines, où la seconde zone de réaction est configurée pour isomériser au moins une partie des oléfines linéaires en oléfines ramifiées, et où la première zone de réaction est opérée à une température comprise entre environ 300 °C et environ 600 °C.

27. La méthode de n'importe quelles des revendications 1 à 24, où l'unité de déshydrogénation-isomérisation comprend une pluralité de zones, et cette pluralité de zones comprend une première zone de réaction et une seconde zone de réaction, où la première zone de réaction est configurée pour déshydrogéner au moins une partie des paraffines en oléfines, où la seconde zone de réaction est configurée pour isomériser au moins une partie des oléfines linéaires en oléfines ramifiées, et où la première zone de réaction est opérée à une température comprise entre environ 350 °C et environ 550 °C.

28. La méthode de n'importe quelles des revendications 1 à 24, où l'unité de déshydrogénation-isomérisation comprend une pluralité de zones, et cette pluralité de zones comprend une première zone de réaction et une seconde zone de réaction, où la première zone de réaction est configurée pour déshydrogéner au moins une partie des paraffines en oléfines, où la seconde zone de réaction est configurée pour isomériser au moins une partie des oléfines linéaires en oléfines ramifiées, et où la première zone de réaction est opérée à une pression totale de réaction comprise entre environ 1.0 atmosphère et environ 5.0 atmosphères.

29. La méthode de n'importe quelles des revendications 1 à 24, où l'unité de déshydrogénation-isomérisation comprend une pluralité de zones, et cette pluralité de zones comprend une première zone de réaction et une seconde zone de réaction, où la première zone de réaction est configurée pour déshydrogéner au moins une partie des paraffines en oléfines, où la seconde zone de réaction est configurée pour isomériser au moins une partie des oléfines linéaires en oléfines ramifiées, et où le temps de permanence d'au moins une partie du premier courant d'hydrocarbures dans la première zone de réaction est tel que le niveau de conversion de paraffines en oléfines est inférieur à environ 50 pour cent en moles.

30. La méthode de n'importe quelles des revendications 1 à 24, où l'unité de déshydrogénation-isomérisation comprend une pluralité de zones, et cette pluralité de zones comprend une première zone de réaction et une seconde zone de réaction, où la première zone de réaction est configurée pour déshydrogéner au moins une partie des paraffines en oléfines, où la seconde zone de réaction est configurée pour isomériser au moins une partie des oléfines linéaires en oléfines ramifiées, et où la seconde zone de réaction est opérée dans un rang de température compris entre environ 200°C et environ 500 °C.

31. La méthode de n'importe quelles des revendications 1 à 24, où l'unité de déshydrogénation-isomérisation comprend une pluralité de zones, et cette pluralité de zones comprend une première zone de réaction et une seconde zone de réaction, où la première zone de réaction est configurée pour déshydrogéner au moins une partie des paraffines en oléfines, où la seconde zone de réaction est configurée pour isomériser au moins une partie des oléfines linéaires en oléfines ramifiées, et où la seconde zone de réaction est opérée avec une pression partielle des hydrocarbures comprise entre environ 0.1 atmosphère et environ 10 atmosphères.

32. La méthode de n'importe quelles des revendications 1 à 24, où l'unité de déshydrogénation-isomérisation comprend une pluralité de zones, et cette pluralité de zones comprend une première zone de réaction et une seconde zone de réaction, où la première zone de réaction est configurée pour déshydrogéner au moins une partie des paraffines en oléfines, où la seconde zone de réaction est configurée pour isomériser au moins une partie des oléfines linéaires en oléfines ramifiées et où la seconde zone de réaction est opérée avec une pression partielle des hydrocarbures comprise entre environ 0.5 atmosphère et environ 2 atmosphères.

33. La méthode de n'importe quelles des revendications 1 à 32, qui comprend en outre introduire au moins une partie du premier courant d'hydrocarbures qui sort de la première zone de réaction dans un échangeur de chaleur, où l'échangeur de chaleur est configurée pour extraire chaleur d'une partie du premier courant d'hydrocarbures avant que celui-ci entre à la seconde zone de réaction.

34. La méthode de n'importe quelles des revendications 1 à 33, où l'unité de déshydrogénation-isomérisation comprend un catalyseur multiple de configuration empilée, où le catalyseur multiple de configuration empilée comprend un catalyseur de la déshydrogénation et un catalyseur de l'isomérisation.

35. La méthode de n'importe quelles des revendications 1 à 28, que comprend en plus introduire hydrogène dans le premier courant d'hydrocarbures.

36. La méthode de n'importe quelles des revendications 1 à 35, que comprend en outre introduire au moins une partie du courant d'hydrocarbures alkyl aromatiques dans une unité de sulfonation, où l'unité de sulfonation est configurée pour sulfoner au moins une partie des hydrocarbures alkyl aromatiques du courant d'hydrocarbures alkyl aromatiques, de manière de produire sulfonates alkyl aromatiques et où au moins une partie des sulfonates alkyl aromatiques produits comprend des sulfonates alkyl aromatiques ramifiés.
